# EUROPEAN PATENT SPECIFICATION

(11) **EP 0 558 656 B1**
(45) Date of publication and mention of the grant of the patent: **17.04.1996**
(21) Application number: 92901632.7
(22) Date of filing: 21.11.1991
(51) Int. Cl.: C07B 31/00, C07B 53/00, C07C 29/136, C07C 29/143, C07C 209/30, C07C 209/44

(54) **NEW METHODS FOR THE CATALYTIC REDUCTION OF ORGANIC SUBSTRATES**
NEUE METHODEN ZUR KATALYTISCHEN REDUKTION ORGANISCHER SUBSTRATE
NOUVEAU PROCEDE DE REDUCTION CATALYTIQUE DE SUBSTRATS ORGANIQUES

(30) Priority: 21.11.1990 US 616892; 13.05.1991 US 698939; 13.05.1991 US 698940; 23.08.1991 US 749111; 14.11.1991 US 792227; 14.11.1991 US 792229; 14.11.1991 US 792233
(43) Date of publication of application: 08.09.1993
(73) Proprietor: MASSACHUSETTS INSTITUTE OF TECHNOLOGY, Cambridge, MA 02139 (US)
(72) Inventor: BUCHWALD, Stephen, L., Somerville, MA 02144 (US); KREUTZER, Kristina, A., Cambridge, MA 02139 (US); WILLOUGHBY, Christopher, A., Somerville, MA 02144 (US); GROSSMAN, Robert, B., Brookline, MA 02146 (US); BERK, Scott, C., Somerville, MA 02144 (US); SPALTENSTEIN, Esther Dutch Village Apartments, Raleigh, NC 27606 (US); GUTIERREZ, Alberto, Rockville, MD 20850 (US)
(74) Representative: Woodcraft, David Charles
(86) International application number: US9108738
(87) International publication number: WO9209545

(56) References cited:
- Chemistry Letters, no. 3, March 1988, The Chemical Society of Japan, T. Nakano et al.: "Bis(n5-cyclopentadienyl)diphenyltitanium-catalyzed hydrosilylation of ketones", pages 481-484, see the whole article
- Bulletin of the Academy of Sciences of the USSR, Division of Chemical Science, vol.3 No.3 pt.2, 20 September 1990, Plenum Publishing Corporation, I P Beletskaya et al.:"Organolanthanides in the catalysis of the hydrosilyation of olefins and ketones", pages 613-614.
- Tetrahedron Letters, vol.2, No.48, 1988, Pergamon Press plc, (GB) M E Jung et al.:"Chirality transfer from silicon to carbon : use of optically pure cyclic silanes with a binaphtalane chiral unit", pages 6199-6202
- Journal of the American Chemical Society, vol. 113, no.13, 19 June 1991, S C Berk et al.:"A catalytic method for the reduction of esters to alcohols", pages 5093-5095
- The Chemistry of Organic Silicon Compounds, pt.2, Chapter 25, 1989, John Wiley & Sons Ltd., Iwao Ojima: "The hydrosilylation reaction", pages 1499-1518

## Description

### Background of the Invention

The present invention relates to processes for catalytically reducing and/or transforming organic carbonyl compounds, and for the catalytic asymmetric or non-asymmetric reduction of ketones, imines, oximes, hydrazones and the like.

Methods currently are known for the catalytic reduction of organic carbonyls. Many such reduction reactions, such as those involving esters, ketones and amides, utilize lithium aluminum hydride or related species as a reducing reagent. Such reagents are quite pyrophoric and can ignite spontaneously upon contact with air or water. Moreover, lithium aluminum hydride typically is dispensed in a volatile liquid such as ether, thus compounding safety concerns. Aside from potential safety issues which surround the use of reducing reagents such as lithium aluminum hydride, their use can be costly as these compounds must be used in stoichiometric rather than catalytic quantities. A further disadvantage of reactions which use lithium aluminum hydride as a reducing agent is that they yield an aluminum salt as a by-product, from which the desired end product is often difficult to isolate.

Chemistry of Organic Silicon Compounds (Part 2, 1989, pages 1499-1518) describes studies on the hydrosilylation of carbonyl compounds and of carbon-nitrogen multiple bonds in reduction of such compounds.

Chemistry Letters 1988(3), pages 481-484 discloses the use of cyclopentadienyl complexes in hydrosilylation of ketones. In particular, bis(cyclopentadienyl/diphenyltitanium was shown to activate on Si-ti bond of diphenylsilane, methylphenylsilane and phenylsilane to bring about the reaction with ketones giving hydrosilylation products in good yields with good selectivity.

Tetrahedron Letters, 29, (1988) pages 6199-6202, describes the use of chiral additives, optically pure binaphthylic cyclic silanes, in the reduction of ketones.

Reactions which reduce organic carbonyls, such as esters, ketones and amides, often are commercially quite significant, as they can be used in the large scale preparation of pharmaceuticals and specialty chemicals. Thus, the safety and economy of the reduction reactions are important considerations. Processes which safely and economically produce amines are of great interest since these compounds are widely used as pharmaceuticals and specialty chemicals. Moreover, many pharmaceutically active amines are optically active compounds, and effective processes for generating amines enriched in a desired enantiomer are thus needed. Currently utilized methods of producing enantiomerically enriched amines rely upon the use of expensive late transition metal catalysts and potentially hazardous reagents.

Accordingly, it would be advantageous to provide safer and more economical processes for reducing organic carbonyl compounds.

It is thus an object of the invention to provide a safer and more economical process for reducing organic carbonyl compounds, including esters, lactones, ketones, amides and imides. Another object is to provide such a reaction where the end product of the reaction is effectively and conveniently isolated. A further object is to provide safe and economical processes for preparing chiral amines, enriched in one enantiomer, by the catalytic asymmetric reduction of imines, oximes, hydrazones, and the like. It is also an object to provide economical and safe non-asymmetric catalytic reduction techniques for imines, oximes, hydrazones and the like. Another object is to provide methods of catalytically reducing ketones to yield alcohol end products of high enantiomeric purity. Other objects will be apparent upon reading the disclosure which follows.

### Summary of the Invention

The invention provides a relatively safe and effective catalytic process for conveniently reducing organic carbonyl compounds, including esters, ketones and amides. The applicability of this process to the manufacture of pharmaceuticals and specialty chemicals will be appreciated by those having ordinary skill in the art. Among the organic carbonyls which can be reduced by the processes of this invention are esters, ketones, amides, and imides. Esters and ketones can be reduced to alcohols, while amides can be reduced to amines. Lactones can be reduced to lactols and/or to diols. In another embodiment of the invention, tertiary amides can be reduced to yield enamine compounds, and imides can be reduced to yield dienamine compounds. A further embodiment of the invention is the catalytic asymmetric reduction of ketones to yield alcohols enriched in one enantiomer, and the catalytic asymmetric reduction of imines, oximes, and the like to yield chiral amines having high enantiomeric purity. The non-asymmetric reduction of imines, and the like, to amines provides another aspect of the invention.

Unless otherwise clear from its context, the term "catalyst" is used interchangeably herein to refer both to the metal complexes or precatalysts before their activation as catalytic species, and to the active catalytic species themselves. Where an achiral precatalyst is used in combination with an optically active additive, the complex added to the reaction mixture is sometimes referred to herein as a "catalyst", even though the actual catalytic entity may not be formed until after activation of the catalyst and/or combination of the chiral additive and the complex.

Generally, the process of the invention involves first generating from a precatalyst an active species of an effective reduction catalyst which is used in the reaction. Where catalytic asymmetric reduction is to occur, the catalyst can be a chiral non-racemic catalyst, enriched in one enantiomer. In one embodiment, the catalyst is a titanium-containing catalyst, however, other catalysts may be used as well.

Activation of the catalyst (where necessary) is effected by subjecting a catalytic amount (i.e., about 3.0 to 10 percent by mole relative to substrate) of the pre-catalyst (e.g., a titanium-containing complex) to between 1 and 2 equivalents of an alkylating or reducing agent, relative to the pre-catalyst. A stoichiometric amount of a silane reducing reagent (relative to substrate) is then combined with the activated catalyst. The desired organic carbonyl substrate is then allowed to react with the silane reagent in the presence of the catalyst.

The reduction of ester, ketone, and imine substrates by this reaction yields a silicon-containing intermediate. Silicon may be cleaved from the intermediate by conventional techniques, after quenching of the catalyst, to yield a crude end product in a more reduced form than the starting compound. The end product may then be purified by known techniques.

Reduction reactions in which the carbonyl substrate is an amide or an imide do not require a silicon cleavage step. Following the reduction of these substrates one need only perform conventional separation and purification techniques to yield the desired end product.

Catalysts suitable for use in practicing this invention are described herein. Included among the suitable catalysts are those which are air stable, and self-activating in the presence of a silane.

Catalytic asymmetric reduction may also be accomplished through another method which involves using an optically active additive in combination with the precatalyst, reductant and substrate. In this embodiment the precatalyst may be chiral or achiral.

In one embodiment, as noted above, a silane compound acts as the reducing agent and the reduction reaction can be carried out in an inert gas such as argon or nitrogen. Alternatively, hydrogen can be used as the reducing agent. In this embodiment, most applicable to the reduction of imines, oximes, and the like, the active catalytic specieis can be generated in the presence of a silane compound and an inert gas. Thereafter, the reduction reaction takes place in the presence of hydrogen which is present in excess and is the stoichiometric reductant. In this embodiment, no silicon cleavage step is required.

### Detailed Description of the Invention

In one embodiment, the process of the invention can be used to catalytically reduce organic carbonyl compounds such as esters, ketones and amides. Esters and ketones can be reduced to alcohols, and where the ester is a lactone, it can be reduced to either a lactol or to a diol. Tertiary amides can be reduced to amines. Tertiary amides having alpha hydrogens can be reduced to enamines, which can be converted to aldehydes by known techniques. Additionally, imides can be reduced to dienamines. One important feature of the process of the invention is that it utilizes relatively inexpensive and safe catalysts and reducing reagents.

A further embodiment of the invention is the catalytic asymmetric reduction of imines, oximes, hydrazones and related compounds such as oxime O-alkyl ethers, oxime O-aryl ethers, N,N-dialkylhydrazones, N,N-diarylhydrazones, and N-alkyl-N-arylhydrazones, to yield amines which are enriched in one enantiomer. These substrates may also be catalytically reduced, non-asymmetrically, to yield amines. The process of the invention is also useful in preparing from ketone substrates, alcohols enriched in one enantiomer.

For catalytic asymmetric reduction reactions, the catalyst can be one which is enriched in one enantiomer. Generally, an enantiomerically enriched catalyst is one which has more than 50 percent of one enantiomer. More specifically, an enantiomerically enriched catalyst is one which preferably has greater than 80%, and most preferably greater than 90%, of one enantiomer.

One important feature of the process of the invention is that relatively inexpensive and safe catalysts and reducing reagents can be used. In addition, some of the catalysts used in the method of the invention are self-activating in the presence of a silane, and need not be maintained in an organic solvent, although the reaction may be carried out in the presence of an organic solvent if desirable. Further, for certain catalysts the reduction reaction can often be carried out in an atmosphere of air, rather than in an inert atmosphere.

The basic steps for the reduction of organic carbonyls according to the invention involve first generating from a precatalyst an active species of an effective catalyst which, depending upon the identity of the catalyst, may be dispensed in an organic solvent such as tetrahydrofuran, ether, toluene, benzene, hexane, or the like. In some cases, it is preferable to maintain this mixture in an atmosphere of an inert gas such as argon or nitrogen within which the reduction reaction takes place. In some instances, especially where certain titanium-containing catalysts are used, as explained below in more detail, the precatalyst is activated by dissolving the catalyst in a solvent together with an alkylating or reducing agent.

Once the active catalyst is formed, it can be mixed with a silane reducing reagent which provides the source of hydride ion for the reduction reaction. In some instances, the catalyst-solvent mixture is maintained at a relatively low temperature (e.g., between about -60°C to -78°C) until it is mixed with the silane. Thereafter, the mixture may be allowed to warm to between about 0°C and room temperature. It is noted, however, that the catalyst may also be generated at room temperature. The organic carbonyl substrate is then reacted, at a temperature between about room temperature and 100°C, with the silane reducing agent in the presence of the activated catalyst. Typically, the reaction requires from about 15 minutes to 48 hours to complete. The reaction can be terminated by deactivating the catalyst through known techniques, such as by exposure to air or by the addition of aqueous sodium hydroxide.

As noted, some of the catalysts are self-activating and can operate in an air atmosphere. In an embodiment using such catalysts, the silane reducing agent, the substrate and the catalyst are all added, sequentially, to a reaction vessel which subsequently is placed in an oil bath preheated to the desired temperature at which the reaction will be conducted.

Where such air-stable catalysts are used, the reduction reaction can be carried out in an air atmosphere or in an atmosphere of an inert gas such as nitrogen or argon. While in many instances the reaction works equally well in either type of atmosphere, it may be preferable (except for some reductions of tertiary amide substrates), at least from a safety standpoint, to carry out the reduction reaction in an ambient atmosphere to avoid the production of silane gas.

The order in which the reactants and catalyst are added to the reaction vessel is not believed to be critical. However, the silane reducing agent, substrate, and catalyst may be added sequentially. Subsequently, the mixture can be heated. Variations of the order in which reactants and catalysts can be added to the reaction vessel are illustrated herein.

In one general procedure for reducing organic carbonyls, the reaction vessel can first be charged with silane reducing agent, substrate and catalyst. The reaction mixture is heated to a temperature at which the reaction is to occur (i.e., 25°C to 100°C) while it is stirred. The reaction is allowed to proceed until all of the substrate is consumed, as may be verified by GLC or TLC analysis of a sample of the reaction mixture. When the substrate is consumed, the reaction mixture is cooled to room temperature. Thereafter, work-up and separation procedures are effected as the reaction mixture is added to a solvent such as tetrahydrofuran (THF). Aqueous sodium hydroxide can then be added to this mixture followed by stirring for 1 to 2 hours. The reaction mixture can then be added to a water/ether mixture, shaken, and separated. The aqueous layer is washed with ether, and the ether extracts are dried over MgSO₄ and concentrated to give the product. This reaction can be conducted in an inert gas such as argon, or in an air atmosphere. The reaction may also be conducted in a flask equipped with a drying tube which includes an agent such as anhydrous CaSO₄.

In a variation of the above general procedure for performing the present reduction reaction, a reaction vessel, such as a Schlenk tube, is heated to a suitable temperature (25° C to 100° C) and is charged with silane reducing agent, substrate, and catalyst, and the reaction mixture is stirred while the desired temperature is maintained. The reaction may also be conducted in a flask equipped with a drying tube which includes an agent such as anhydrous CaSO₄. This method is generally well suited to reactions which will be conducted in an air atmosphere.

The reduction, when performed with esters and ketones, as well as with imines, oximes, hydrazones and the like, yields a silicon-containing intermediate compound. The silicon may be cleaved from the intermediate by hydrolysis or alcoholysis, and a variety of known extraction techniques may be used to isolate the desired end product of the reduction reaction. For example, silicon cleavage may be effected by treatment with alcoholic or aqueous solutions (or mixed alcohol/aqueous solutions) of acids or bases such as hydrochloric acid or sodium hydroxide. Alternatively, silicon cleavage may be effected by the addition of an alcohol, such as methanol or ethanol, or a mixed alcohol/water solution. Subsequently, extraction, separation and drying techniques can be utilized to recover the crude product, which scan then be purified if necessary by a conventional technique such as chromatography. The reductions of tertiary amides, imides and the reduction of imines utilizing hydrogen as the stoichiometric reductant do not require a silicon cleavage step. However, separation and purification techniques generally must be effected to recover the desired end product.

The invention is generally applicable to the reduction of organic carbonyl substrates. Exemplary carbonyl compounds include acyclic and cyclic esters, ketones and amides. The invention is also potentially applicable to the reduction of compounds such as aldehydes, acids, acid chlorides, nitriles and thioesters. Ketones may also be reduced by catalytic asymmetric techniques.

Substrates which may be catalytically asymmetrically reduced to chiral amines include imines, oximes, hydrazones, oxime O-alkyl ethers, oxime O-aryl ethers, N,N-dialkylhydrazones, N,N-diarylhydrazones, and N-alkyl-N-arylhydrazones. As noted above, these substrates may also be catalytically reduced non-asymmetrically, to yield racemic amines.

A variety of catalysts can be used effectively in the reduction reactions of the present invention. Exemplary catalysts broadly include those which consist of a metal of group 4, 5 or 6, a lanthanide or an actinide, which: a) is in less than its maximum oxidation state or is capable of being converted to a complex in less than its maximum oxidation state; and/or b) is a metal hydride; and/or c) is capable of being converted to a metal hydride. Examples of group 4, 5 and 6 metals which may be useful in the present invention include titanium, vanadium and chromium.

Preferred catalysts are titanium-containing catalysts such as bis (trimethylphosphine) titanocene, titanocene monochloride and titanocene dichloride. Of the above identified titanium-containing catalysts, titanocene dichloride and titanocene monochloride are activated by reaction with an alkylating or reducing agent. Examples of other suitable titanium-containing catalysts include compounds having the following general structures: L(L')(L")Ti; L(L')(L")(L''')Ti; L(L')Ti-X; L(L')(L")Ti-X; L(L')TiX₂; L(L')TiH; and L(L')(L")TiH, where X is a halogen, and where L, L', L" and L''' can be some combination of -OR, -SR, -NR(R'), R, Si(R)(R')(R"), and P(R)(R')(R") (where R, R' and R" may be an alkyl, aryl, hydride or silyl group and may be different or the same), or a cyclopentadienyl group (hereinafter "Cp") having the formula 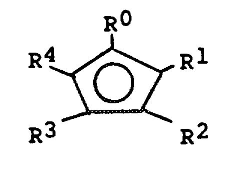 where R⁰, R¹, R, R³ and R⁴ may be hydrogen, alkyl, aryl, trialkylsilyl, triarylsilyl,
(dialkyl)arylsilyl, or (diaryl)alkylsilyl groups in any combination and may all be the same or different. More specific examples of such compounds include titanocene alkoxides, titanocene aryloxides, titanocene (III) hydrides, titanocene (aryloxy) chlorides, titanocene (alkoxy) chlorides, titanocene (alkyl) alkoxides, titanocene (alkyl) aryloxides, titanocene (aryl) alkoxides, and titanocene (aryl) aryloxides.

As noted above, one preferred titanium-containing catalyst is bis (trimethylphosphine) titanocene, having the formula

Cp₂Ti(PMe₃)₂ (I)

Wherein Cp represents a η⁵-cyclopentadienyl group and Me represents a methyl group.

The catalytic species identified above by formula I is self-activating and should be maintained in solution with an organic solvent and maintained at a relatively low temperature (0°C to 80°C) in the absence of air and excess moisture.

Another preferred titanium-containing catalyst is titanocene dichloride which is represented by the formula

Cp₂TiCl₂

wherein Cp represents a η⁵-cyclopentadienyl group. Alternatively, titanocene monochloride (Cp₂TiCl) may be used as a catalyst as well.

Another preferred titanium-containing catalyst has the general formula

Cp₂Ti(OR)(OR')

where R and R' can be alkyl, aryl, silyl or hydrogen and can be the same or different.

A catalyst which is particularly useful in conducting catalytic asymmetric reduction reactions is generally represented by the formula

YTiX₂

where Y represents ethylene-1,2-bis (η⁵-4,5,6,7-tetrahydroindenyl), and X represents groups including halides, alkoxides, amides, sulfides, alkyls, aryls, hydrides, phosphines and tri-substituted silyls. Catalysts having the ethylene-1,2-bis(η⁵-4,5,6,7-tetra-hydroindenyl) backbone are referred to herein as "BIE" catalysts. In one preferred catalyst X₂ represents 1,1'-binaphth-2,2'-diolate. Specific preferred catalysts for asymmetric reduction thus include (R,R)-Ethylene-1,2-bis- (η⁵-4,5,6,7-tetrahydroindenyl)titanium (R)-1,1'-binaphth-2, 2'-diolate and (S,S)-Ethylene-1,2-bis(η⁵-4,5,6,7-tetrahydroindenyl)- titanium (S)-1,1'-binaphth-2, 2'-diolate.

The BIE catalysts, when used in catalytic asymmetric reduction reactions, should be enriched in one enantiomer of the molecule. Generally, enantiomeric enrichment requires more than 50% of one enantiomer, but more specifically requires more than 80% of one enantiomer. In a preferred embodiment, an enantiomerically enriched catalyst has more than 90% of one enantiomer. Of course, the greater the level of enantiomeric purity of the catalyst, the greater will be the enantiomeric purity of the end product of the reaction.

A variety of self-activating, air-stable precatalysts can also be used effectively in the reduction reactions of the present invention. Exemplary self-activating, air-stable catalysts broadly include those having the general formulas:
M(L)(L')(L")
M(L)(L')(L")(L''')
M(L)(L')(L")(L''')(L^{iV})
M(L)(L')(L")(L''')(L^{iV})(L^{V})
where M is a group 3, 4, 5 or 6 metal, a lanthanide, or an actinide and where L, L', L", and L''', L^{iV} and L^{V}, independently, can be some combination of H, an alkyl group, an aryl group, a halogen, Si(R)(R')(R"), -OR, -SR, or -NR(R'), P(R)(R')(R"), where R, R', and R" may be H or an alkyl, aryl, or silyl group and may be different or the same. Examples of group 3, 4, 5 or 6 metals which may be useful with the present invention include titanium, yttrium, niobium, vanadium and chromium. Examples of useful lanthanides include samarium, ytterbium, and lutetium. Examples of useful actinides include thorium and uranium. Titanium, however, is the most preferred metal.

Among the catalysts generally identified above, the most preferred self-activating, air-stable catalysts include metal alkoxides and metal aryloxides such as titanium (IV) alkoxides and titanium (IV) aryloxides. Specific catalysts include titanium (IV) isopropoxide, titanium (IV) ethoxide, trichlorotitanium (IV) isopropoxide, titanium (IV) methoxide, and titanium (IV) butoxide.

Currently, the most preferred self-activating, air stable catalysts include titanium (IV) isopropoxide, titanium (IV) ethoxide and trichlorotitanium (IV) isopropoxide.

The air-stable, self-activating catalysts are present in the reaction in catalytic quantities, ranging from about 5-10 mole percent, relative to the substrate.

The catalysts useful in this invention may be active as electronically neutral molecules, anions or cations.

The titanocene dichloride, titanocene monochloride and BIE catalysts must be activated by reaction with an alkylating agent or reducing agent, preferably in an organic solvent. Suitable alkylating and reducing agents are known to those skilled in the art and generally include organometallic compounds. Examples of such compounds include alkylmagnesium halides, alkyllithium compounds, alkylaluminum compounds and boron, aluminum, or other metal hydrides. Particularly preferred alkylating agents include n-pentylmagnesium bromide, n-butyllithium, and sodium acetylide. Preferred reducing agents include sodium bis(2-methoxyethoxy)aluminum hydride (Red Al®). Preferably, about 100 to 200% by mole of the alkylating or reducing agent relative to the catalyst should be reacted with the catalyst in order for activation to occur. More preferably, titanocene dichloride requires about 200% by mole, relative to the catalyst, of alkylating agent while titanocene monochloride requires about 100% by mole relative to the catalyst. The activation of such catalysts by reaction with an alkylating agent is further described and illustrated in the examples.

One skilled in the art will appreciate that a variety of solvents can be used with these catalysts. One general requirement of a suitable solvent is that the catalyst must be completely or partially soluble within the solvent. Complete solubility is not required as there need only be enough catalyst present in the solution to facilitate a reaction. Exemplary solvents include tetrahydrofuran, toluene, benzene, hexane, ether and the like. An additional advantage of the invention is that the substrate may be present in the organic solvent at relatively high concentrations (e.g., about lM), thus enabling smaller reactors to be used and less waste solvent to be generated. It is noted that no solvent other than the silane itself may be required.

As noted above, the reducing reagent preferred in the present processes is a silane compound which must be capable of supplying a hydride ion during the reduction reaction. Exemplary silane compounds which may be used in these processes are represented by the formulas shown below.

R(R')SiH₂ (II)

RSiH₃ (III)

RO(R'O)SiH₂ (IV)

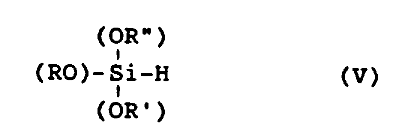

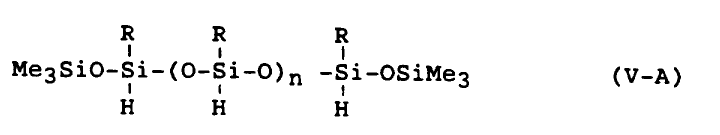

where R, R' and R" represent hydride, alkyl or aryl groups and may be the same or different. Specific examples of suitable silane reducing reagents include silane, diphenylsilane, phenylsilane, diethylsilane, dimethylsilane and triethoxysilane, trimethoxysilane, and poly(methylhydrosiloxane).

Preferably, the silane compound, when used as the reducing reagent, is present in an amount ranging from about 100 to about 300% by mole as compared to the amount of the substrate. Where the reducing agent is hydrogen the silane can be present at about 0.1 to 5 equivalents, and more preferably 0.1 to 2.5 equivalents, relative to the catalyst.

In one aspect of the invention, amides may be reduced to cyclic or acyclic enamine compounds having the general formula 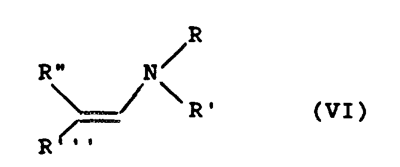 where R, R', R" and R''' represent hydrogen, alkyl, or aryl groups. The enamines produced may also include cyclic compounds where R and R", shown above in formula VI, are connected. It is believed that in this reaction the enamines may sometimes be isolable intermediates in the reduction reactions of heterocyclic amides to heterocyclic amines.

The invention, as noted above, also involves the catalytic asymmetric reduction of certain substrates to yield end products having a high degree of enantiomeric purity. Substrates such as imines, oximes, hydrazones, and the like yield amines having a high degree of enantiomeric purity. In addition, ketone substrates can be reduced to yield alcohols having a high degree of enantiomeric purity. The desired substrate can be reduced to an end product enriched in one enantiomer using a suitable, enantiomerically enriched catalyst of the type described above. A preferred catalyst is one which is enriched in (R,R)-Ethylene-1,2-bis(η5-4,5,6,7-tetrahydroindenyl) titanium (R)-1,1'-binaphth-2,2'-diolate. Preferably, this catalyst contains at least about 80% of the (R,R,R) enantiomer. Another preferred catalyst is one which is enriched in (S,S)-Ethylene-1,2-bis(η⁵-4,5,6,7-tetrahydroindenyl) titanium (S)-1,1'-binaphth-2,2'-diolate. Suitable catalysts for effecting this method of catalytic asymmetric reduction contain at least about 80% of the (S,S,S) enantiomer.

The mechanism of the asymmetric reduction of ketones by BIE-based catalysts is believed to be as follows. First, the reaction requires the initial binding of the oxygen of the ketone substrate to the metal of the catalyst, followed by the migration of the hydride to the carbonyl group of the substrate. Since the carbonyl binds through one of the lone pairs of the oxygen atom, it must be coplanar with the hydride. For the hydride migration to occur, however, the carbonyl must turn to present a face of the double bond, and in doing so, it must place its substituents out of the plane. In the achiral catalyst this migration can occur over either enantiotopic face, but when a chiral catalyst is used the ketone preferentially places the large substituent in a less crowded environment. The reaction is therefore expected to occur through the less hindered transition state, resulting in an asymmetric hydrosilylation.

The degree of enantiomeric excess ("ee") for the amine or alcohol reaction product depends on a number of factors including the enantiomeric purity of the catalyst, the specific compound being reduced, and reaction conditions. For many compounds produced through this reaction relatively high enantiomeric excess values are obtained. In some instances, the "ee" exceeds 90%.

In addition to the procedure described above, optically active end products may be produced by the alternative procedure described below. According to this embodiment an optically active additive is combined with a metal alkoxide or metal aryloxide precatalyst of the type described above. The silane and the desired substrate may then be added, together with an inert solvent. The reaction is then commenced either in an inert gas (if the silane serves as the reducing agent) or in a hydrogen atmosphere (if hydrogen serves as the reducing agent). The reaction may be carried out at a temperature ranging from about 25°C to about 100°C.

Suitable optically active additives include amines, diamines, alcohols, diols, acids, diacids thiols and phosphines. Exemplary compounds include (1R, 2R)-diaminocyclohexane; (1S,2S)-diaminocyclohexane; (R)-1, 1'-Bi-2-naphthol, (S) 1, 1'-Bi-2-naphthol; (1R, 2S)-ephedrine; (1S, 2R)-ephedrine; 1,1,4,4-tetraphenyl-2, 3-0-isopropylidene -D-threitol.

This alternative procedure for producing optically active end products can be effected in the following manner. Activation of the catalyst (if necessary) proceeds according to the method previously described. Thereafter, approximately 0.1 to 10 mole % of catalyst relative to substrate is combined with about 0.1 to 100 mole % of the chiral additive relative to substrate in an inert solvent. The silane is then added to the mixture at 100 to 300 mole % relative to substrate, followed by the addition of the substrate. The mixture may then be reacted, at a temperature between about 25°C to 100°C. This reaction may be carried out in an inert gas such as argon or nitrogen, using the silane compound as the reducing agent. Where imine, oxime, and hydrazone substrates are involved, the reaction may alternatively be carried out in hydrogen, using hydrogen as the reducing agent.

The order in which the catalyst and reactants are combined is not believed to be critical. The chiral additive and silane reductant may be combined first followed by the addition of catalyst and then substrate. Also, the catalyst and silane reductant may be combined first, followed by the addition of chiral additive and then substrate. The catalyst and chiral additive may also be combined first, followed by the addition of silane reductant and then substrate. The reaction may or may not be heated at any stage in the process of combining the reagents and substrate.

The non-asymmetric reduction of imine, oxime, hydrazone, oxime O-alkyl ethers, oxime O-aryl ethers, N,N-dialkylhydrazone, N,N-diarylhydrazone, and N-alkyl-N-arylhydrazone substrates proceeds generally as described above with respect to the catalytic reduction of carbonyls. Preferably, an achiral catalyst is used to effect such a reduction reaction. This reduction reaction is further described in the examples which follow.

In the above description, the mole percent is relative to the amount of substrate unless otherwise noted. Moreover, one skilled in the art will understand that inert solvents include, by way of example, tetrahydrofuran, toluene, hexane, benzene, and ether.

The invention is further illustrated by the examples which follow.

### Example 1 (Reduction of ethyl cyclohexylcarboxylate to cyclohexylmethanol)

To a dry Schlenk tube under argon was added 37 mg of titanocene dichloride (0.15 mmol) and 2 mL of tetrahydrofuran. The slurry was cooled to about -78°C in a dry ice/acetone bath, and a 1.6M hexane solution of n-butyllithium (188 µL, 0.3 mmol) was added. The reaction mixture changed color from red to dark brown. After stirring for 15 minutes, 1.4 mL of triethoxysilane (7.5 mmol) and 468 mg of ethyl cyclohexylcarboxylate (3.0 mmol) were added and the reaction mixture was allowed to warm to room temperature. After 1 hour, the catalyst was deactivated by exposure to air until the color changed from dark brown to yellow. Next, 10mL of tetrahydrofuran and 0.5 mL of concentrated HCl were added. After stirring for 2 hours, the mixture was added to a water/ether mixture (150 mL each), shaken vigorously, and the layers were then separated. The aqueous layer was extracted with ether (2x50 mL), the combined organic extracts were washed with brine, dried over MgSO₄, filtered, and concentrated by rotary evaporation. The crude product was purified by flash chromatography (ether:hexane = 2:3), which afforded 274 mg (80% yield) of cyclohexylmethanol.

### Example 2-A (Reduction of ethyl 2-phenylethanoate to phenethyl alcohol)

To a dry Schlenk tube under argon was added 50 mg of titanocene dichloride (0.2 mmol) and 2 mL of tetrahydrofuran. The slurry was cooled to -78°C in a dry ice/acetone bath and 200 µL of a 2M ether solution of pentylmagnesium bromide (0.4 mmol) was added. After stirring for 15 minutes, 930 µL of diphenylsilane (5.0 mmol) was added and the reaction mixture was allowed to warm to 0°C. Next 637 µL of ethyl 2-phenylethanoate (4.0 mmol) was then added and the reaction mixture was allowed to warm to room temperature. After 2 hours, an additional 200 µL of diphenylsilane was added and the reaction was stirred for 1.5 hours. The catalyst was then quenched by exposure to air until the color changed from dark brown to yellow. 5 mL of tetrahydrofuran and 15 mL of 1N NaOH in MeOH solution were then added. After stirring for 2 hours, the mixture was added to a brine/hexane mixture (150 mL each), shaken vigorously, and the layers were then separated. The aqueous layer was extracted with hexane (2x50 mL), the combined organic extracts were dried over MgSO₄, filtered, and concentrated by rotary evaporation. The crude product was purified by flash chromatography (ether:hexane = 3:7), which afforded 397 mg (81% yield) of phenethyl alcohol.

### Example 2-B (Alternative method of reducing ethyl 2-phenylethanoate to phenethyl alcohol)

To a dry Schlenk tube under argon was added 50 mg of titanocene dichloride (0.2 mmol) and 2 mL of tetrahydrofuran. The slurry was cooled to -78°C in a dry ice/acetone bath and 250 µL of a 1.6M hexane solution of n-butyllithium (0.4 mmol) was added. After stirring for 15 minutes, 1.8 mL of triethoxysilane (10 mmol) was added, followed by 500 µL ethyl 2-phenylethanoate (3.0 mmol), and the reaction mixture was allowed to warm to room temperature. The reaction mixture bubbled vigorously. When the bubbles subsided, the reaction mixture began to warm rapidly, causing tetrahydrofuran to begin refluxing. When the mixture cooled back to room temperature, the catalyst was quenched by exposure to air until the color changed from dark brown to yellow. Next, 5 mL of tetrahydrofuran, 15 mL of EtOH, and 0.6g of NaOH (15 mmol) were then added. After stirring for 2 hours, the mixture was added to a water/ether mixture (150 mL each), shaken vigorously, and the layers were then separated. The aqueous layer was extracted with ether (2x50 mL), the combined organic extracts were dried over MgSO₄, filtered, and concentrated by rotary evaporation. The crude product was purified by flash Chromatography (ether:hexane =1:1), which afforded 300 mg (82% yield) of phenethyl alcohol.

### Example 3 (Reduction of ethyl 3-phenylpropionate to 3-phenylpropyl alcohol)

To a dry Schlenk tube under argon was added 50 mg of titanocene dichloride (0.2 mmol) and 2 mL of tetrahydrofuran. The slurry was cooled to -78°C in a dry ice/ acetone bath and 250 µL of a 1.6M hexane solution of n-butyllithium (0.4mmol) was added. After stirring for 15 minutes, 930 µL diphenylsilane (5.0 mmol) was added and the reaction mixture was allowed to warm to 0°C. Then 712 µL of ethyl 3-phenylpropionate (4.0 mmol) was added, and the reaction mixture was allowed to warm to room temperature. After 0.5 hour, the catalyst was deactivated by exposure to air until the color changed from dark brown to yellow. 5 mL of tetrahydrofuran and 15 mL of a solution of 1N NaOH in MeOH were then added. After stirring for 2 hours, the mixture was added to a brine/hexane mixture (150 mL each), shaken vigorously, and the layers were then separated. The aqueous layer was extracted with hexane twice (2x50mL), the combined organic extracts were dried over MgSO₄, filtered, and concentrated by rotary evaporation. The crude product was purified by flash chromatography (ether:hexane = 2:3), which afforded 446 mg (82% yield ) of 3-phenylpropyl alcohol.

### Example 4 (Reduction of N-phenylpyrrolidinone to N-phenylpyrrolidine)

Titanocene dichloride (0.55g, 2.2 mmol) was dissolved in 15 mL of tetrahydrofuran in a nitrogen atmosphere and the solution was cooled to -78°C. Next, 2.8 mL of n-butyl lithium (1.6M) was added to the solution. The reaction mixture was stirred for 15 min and then 9.0 mL of diphenylsilane (49 mmol) was added. The mixture was warmed to room temperature and 3.6g of N-phenylpyrrolidinone (22 mmol) was added to the black solution, which began bubbling and became warm after 1-2 minutes. The reaction mixture was stirred overnight at room temperature and then heated to 55°C for 1 hour. The solution was then poured into 50 mL of ethyl ether and extracted with 1M HCl (5 x 50 mL). The aqueous layer was washed with 50 mL of ethyl ether and a saturated solution of aqueous NaHCO₃ was added to the aqueous layer until it became basic to pH paper. The solution was then extracted with ethyl ether (5 x 50 mL). An emulsion formed and 50 mL of a saturated NaCl solution was added to the ether and emulsion layer. The ether was decanted away from the emulsion, which was extracted with 75 mL of ethyl ether. The ether solution was then dried over MgSO₄, filtered, and evaporated in vacuo. A yellow-orange oil (3.5 g, 86% pure by GC, 93% crude yield) was obtained. The ¹H NMR spectrum showed that the impurity was a siloxane byproduct from the reaction. The product was again dissolved in ether, extracted into aqueous HCl, and extracted back into ether after neutralizing the acid with NaHCO₃. After drying over MgSO₄, filtration, and concentration in vacuo, 2.3g of N-phenylpyrrolidine as a yellow oil was isolated (72% isolated yield).

### Example 5 (Reduction of N-benzylpyrrolidinone to N-benzylpyrrolidine)

Titanocene dichloride (0.28g, 1.1 mmol) was dissolved in 15 mL of tetrahydrofuran in a nitrogen atmosphere, and the reaction mixture was cooled to -78°C, and 2.4 mL of a 1.6M solution of n-butyllithium in hexane was added. The reaction mixture was stirred for 15 min and then 4.5 mL diphenylsilane (24 mmol) was added. After warming the reaction mixture to room temperature, 1.8g of N-benzylpyrrolidinone (11 mmol) was added to the black solution, which began bubbling and became warm (i.e., about 30°C) after 1-2 min. The reaction mixture was stirred overnight at room temperature and then heated to 55°C for 1 hour. The solution was then poured into 50 mL of ethyl ether and extracted with 1 M HCl (3 x 50 mL). The aqueous layer was washed with ether (2 x 25 mL) and then neutralized with a 5 M NaOH solution so that the aqueous layer was basic to pH paper. The product was extracted into ether (3 x 50 mL) and the emulsion that formed was also extracted with ether (50 mL). The combined ether extracts were dried over MgSO₄, filtered, and concentrated in vacuo. N-benzylpyrrolidine was isolated as a yellow oil (1.58 g of 95% pure material, 84% yield).

### Example 6 (Reduction of acetophenone to secphenethyl alcohol)

To a dry Schlenk tube under argon was added 37.6 mg (0.15 mmol) of titanocene dichloride and 3 mL of tetrahydrofuran. The slurry was cooled to -78°C and a 1.64 M hexane solution of n-butyllithium (190 µL, 0.3 mmol) was added. After stirring for 15 minutes, triethoxysilane (140 µL, 0.75 mmol) was added. The black mixture was allowed to warm to 0°C, then acetophenone (350 µL, 3.0 mmol), followed by more triethoxysilane (700 µL, 3.75 mmol) were added. The reaction mixture was allowed to stir for 6 hours at room temperature. The Schlenk tube was opened to air and 5 mL of water were added and allowed to stir at room temperature for 1 hour. A solution of 5 M NaOH (1 mL) was added and the mixture was allowed to stir. When the reaction mixture had turned to a white slurry additional 5M NaOH and 5 mL of tetrahydrofuran were added. After stirring for 1 hour, the mixture was added to a water/ether mixture (150 mL each), shaken vigorously, and the layers were then separated. The aqueous layer was extracted with ether (2x50mL) and the combined organic extracts were dried over MgSO₄, filtered, and concentrated by rotary evaporation. The crude product was purified by flash chromatography (ether:hexane = 1:2), which yielded 295mg (80% yield) of sec-phenethyl alcohol.

### Example 7 Reduction of acetophenone N-ethyl imine to N-ethyl-1-phenylethylamine

Titanocene dichloride (50 mg, 0.20 mmol) was dissolved in 2 mL of dry toluene in a Schlenk tube under a nitrogen atmosphere. The mixture was cooled to -78°C and a solution of n-butyllithium (250 µL, 0.40 mmol, 1.6 M in hexane) was added. The mixture was warmed to 0°C and stirred until it turned a dark brown color. Phenylsilane (370 µL, 3.0 mmol) was added and the reaction mixture turned blue, then brown, in color. After warming to room temperature, acetophenone N-ethyl imine (294 mg, 2.0 mmol) was added and the mixture was heated to 77°C. After 30 min. the reaction mixture was cooled to room temperature, diluted with 30 mL of ether and 3 mL of 1 M NaOH in methanol and allowed to stir for 1 hour. The resulting mixture was then washed with 20 mL of water and extracted with 1 M HCℓ (2 x 20 mL). The aqueous layer was separated, basified with 5 M NaOH (until strongly basic to pH paper), and extracted with ether (3 x 30 mL). The ether solution was dried over anhydrous sodium sulfate and concentrated to give 224 mg (1.5 mmol, 75% yield) of N-ethyl-l-phenylethylamine as a yellow oil.

### Example 8 Asymmetric Reduction of acetophenone N-methyl imine to N-methyl-1-phenylethylamine

(R,R)-Ethylene-1,2-bis(η⁵-4,5,6,7-tetrahydroindenyl) titanium (R)-1,1'-binaphth-2,2'-diolate (53 mg, 0.089 mmol) was dissolved in 4 mL of dry benzene in a Schlenk tube under a nitrogen atmosphere. The tube was wrapped in aluminum foil. A solution of n-butyllithium (72 µL, 0.12 mmol, 1.6 M in hexane) was added and the mixture was shaken until it turned a dark red brown color. After 5 minutes phenylsilane (164 µL, 1.33 mmol) was added and the reaction mixture turned blue, then brown, in color. After 10 minutes acetophenone N-methyl imine (119 mg, 0.89 mmol) was added. The tube was shaken and left for about 20 hours at room temperature. The resulting solution was diluted with 15 mL of ether and 3 mL of methanol and allowed to stir for 2 hours. The reaction mixture was then washed with 10 mL of water and extracted with 1 M HCℓ (2 x 10 mL). The aqueous layer was separated, basified with 5 M NaOH (until strongly basic to pH paper), and extracted with ether (3 x 20 mL). The ether solution was dried over anhydrous sodium sulfate and concentrated to yield 60 mg (0.45 mmol, 50% yield) of N-methyl-1-phenylethylamine as a yellow oil. The yield for repeated experiments ranged from 50 to 80%. The optical rotation showed that the (R)-(+)-enantiomer was obtained.

The enantiomeric excess of the amine so isolated was determined by dissolving the amine (10 mg, 0.074 mmol) in 600 µL CDCℓ₃, and then adding triethylamine (19 µL, 0.14 mmol) followed by (S)-α-methoxy-α-(trifluoromethyl)phenylacetyl chloride (16 µL, 0.083 mmol). Integration of the N-methyl peaks in the ¹H NMR spectrum showed a 97% diastereomeric excess for the amide (the peaks were compared to those in the spectrum of the amide prepared in the same manner by the reaction of racemic amine and (S)-α-methoxy-α-(trifluoromethyl) phenylacetyl chloride). The diastereomeric excess for repeated experiments ranged from 97-99%. This indicates that the enantiomeric excess of the amine produced in this procedure is 97-99%.

### Example 9 Asymmetric Reduction of indanone N-methyl imine to N-methyl-1-indanamine

(R,R)-Ethylene-1,2-bis(η⁵-4,5,6,7-tetrahydroindenyl) titanium (R)-1,1'-binaphth-2,2'-diolate (30 mg, 0.050 mmol) was dissolved in 4 mL of dry benzene in a Schlenk tube under a nitrogen atmosphere. The tube was wrapped in aluminum foil. A solution of n-butyllithium (41 µL, 0.065 mmol, 1.6 M in hexane) was added and the mixture was shaken until it turned a dark red brown color. After 5 min. phenylsilane (93 µL, 0.75 mmol) was added and the reaction mixture turned blue, then brown, in color. After 10 minutes indanone N-methyl imine (72.6 mg, 0.50 mmol) was added. The tube was shaken and left for about 20 hours at room temperature. The resulting solution was diluted with 15 mL of ether and 3 mL of methanol and allowed to stir for 2 hours. The reaction mixture was then washed with 20 mL of water and extracted with 1 M HCℓ (2 x 20 mL). The aqueous layer was separated, basified with 5 M NaOH (until strongly basic to pH paper), and extracted with ether (2 x 50 mL). The ether solution was dried over anhydrous sodium sulfate and concentrated to yield 65 mg (0.44 mmol, 88% yield) of N-methyl-1-indanamine as a yellow oil. The (-) - enantiomer was obtained.

The enantiomeric excess of the amine so isolated was determined by dissolving the amine (10 mg, 0.074 mmol) in 600 µL CDCℓ₃, and then adding triethylamine (19 µL, 0.14 mmol) followed by (S)-α-methoxy-α-(trifluoromethyl)phenylacetyl chloride (16 µL, 0.083 mmol). Integration of the proton peaks in the ¹H NMR spectrum showed a 70% diastereomeric excess for the amide (the peaks were compared to those in the spectrum of the amide prepared in the same manner from racemic amine and (S)-α-methoxy-α-(trifluoromethyl) phenylacetyl chloride). This indicates that the enantiomeric excess of the amine produced in this procedure is 70%.

### Example 10: Asymmetric Reduction of Acetophenone to 1-phenylethanol.

Titanium (IV) isopropoxide (30µL, 0.10 mmol) was added to a solution of (R,R)-1,2-bis(benzylamino)-cyclohexane (29µL, 0.10 mmol) and triethoxysilane (370µL, 2.00 mmol) in THF (6 mL) under an argon atmosphere, followed by rinsing with THF (1 mL). After 30 minutes at room temperature, the mixture was heated rapidly to the reflux temperature, then allowed to cool slowly to 45°C. The mixture turned a dark bluish color. Then acetophenone (118µL, 1.01 mmol was added. The mixture gradually decolorized, then returned to the dark color. After 16 hours the reaction mixture was quenched with 15% NaOH (4mL). The mixture was diluted with THF and water and allowed to stir vigorously. It became colorless. Five hours later, GC on a poly(phenylmethylsiloxane) column showed the mixture consisted of 96% 1-phenylethanol and 2% acetophenone (the proportion of the diamine was not determined), and GC on a chiral Cyclodex B column showed that the alcohol had an ee of 8% in favor of the (S) enantiomer. The reaction mixture was diluted with ether, and the organic layer was washed with a mixture of 1 N HCl and brine, dried over MgSO₄, evaporated, and dried in vacuo to give 1-phenylethanol, pure by ¹H NMR, in 73% yield.

### Example 11: Asymmetric Reduction of Acetophenone to 1-phenylethanol-

A mixture of titanium (IV) isopropoxide (15µL, 0.05 mmol) and triethoxysilane (650 µL, 3.50 mmol) in THF (6 mL) was warmed to 46°C. Then (R,R)-1,2-bis(benzylamino)cyclohexane (300µL, 1.03 mmol) was added. A small amount of bubbling occurred. After 12 minutes acetophenone (118 µL, 1.01 mmol) was added, and the bubbling ceased. The reaction mixture turned yellow over a period of hours. After 24 hours reaction time, the mixture was quenched as described above. GC of the reaction mixture showed that all the acetophenone had been consumed, and GC on a chiral column showed that the product, 1-phenylethanol, had an ee of 37% in favor of the (S) enantiomer.

### Example 12: Asymmetric Reduction of Acetophenone to 1-phenylethanol-

A mixture of titanium (IV) isopropoxide (90µL, 0.3 mmol) and 1,1,4,4-tetraphenyl-2,3-0-Isopropylidene-D-threitol (280 mg, 0.06 mmol) in THF (5 mL) under an argon atmosphere was warmed to 45°C. Then triethoxysilane (1.1 mL, 6 mmol) was added dropwise. The solution bubbled profusely. After 30 minutes the bubbling had subsided and acetophenone (350 µL, 3 mmol) was added. After 24 hours the reaction was quenched by adding THF (5 mL) and 1 N NaOH (15 mL) and allowed to stir for one hour. The mixture was then diluted with water and ether (75 ml each) and shaken vigorously. GC on a poly(phenylmethylsiloxane) column showed the compound was greater than 95% 1-phenylethanol, and GC on a chiral Cyclodex B column showed that the alcohol had an ee of 33.6% in favor of the (S) enantiomer. The ether layer was collected and dried over MgSO₄ to afford a milky liquid composed of a mixture of 1-phenylethanol and 1,1,4,4-tetraphenyl-2,3-O-Isopropylidene-D-threitol. This was diluted with pentane, filtered and evaporated to obtain 1-phenylethanol in 62% yield.

### Experimental Procedure for the Titanium Alkoxide - Catalyzed Reduction of Esters to Alcohols

General Procedure I: A dry flask equipped with a drying tube (anhydrous CaSO₄) and a magnetic stir bar was immersed in a 40°C oil bath and charged with triethoxysilane (1.38 mL, 7.5 mmol) and the ester (3 mmol). Then, titanium (IV) isopropoxide (45 µL, 0.15 mmol) was added, and the reaction mixture was stirred at 40°C until GLC analysis of an aliquot taken from it showed complete disappearance of the ester (i.e., about 4 to 18 hours). The reaction mixture was cooled to room temperature and added to THF (7 mL). Aqueous NaOH (1 N, 15 mL) was then added to the solution. After vigorous stirring at room temperature for 1-2 hours, the reaction mixture was added to a water/ether mixture (50 mL each), shaken vigorously, and separated. The aqueous layer was washed with an additional 50 mL of ether, and the combined ether extracts were dried over MgSO₄. Concentration in vacuo afforded the product (often greater than 95% purity as estimated by NMR or GC).

### Example 13 (Reduction of ethyl decanoate)

General procedure I was followed to reduce ethyl decanoate (696 µL, 3 mmol). The reduction took 6.5 hours at 40°C. Work-up yielded 441 mg (93% yield) of decanol as a clear oil.

### Example 13-A (Reduction of ethyl decanoate)

A dry Schlenk tube under argon was charged with titanium (IV) ethoxide (32 µL, 0.15 mmol) and triethoxysilane (1.39 mL, 7.5 mmol) and heated to 40°C. After 15 min., ethyl decanoate (696 µL, 3 mmol) was added. After 18 hours, the reaction was determined to be complete by GC analysis. Standard work-up afforded 444 mg (93% yield) of decanol as a clear oil.

### Example 14 (Reduction of ethyl 6-bromohexanoate)

General procedure I was followed to reduce ethyl 6-bromohexanoate (553 µL, 3.1 mmol). The reduction took 4 hours at 40°C. Work-up yielded 525 mg (97% yield) of 6-bromohexanol as a clear oil.

### Example 15 (Reduction of methyl 10-undecenoate)

General procedure I was followed to reduce methyl 10-undecenoate (594 mg, 3 mmol). The reduction took 9 hours at 40°C. Work-up yielded 431 mg (85% yield) of 10-undecen-1-ol as a clear oil.

### Example 16 (Preparation of 4-(2-phenylethenyl) morpholine)

4-(2-phenylacetyl)morpholine (0.410 g, 2.0 mmol) was dissolved in C₆H₆ (5 mL) under a nitrogen atmosphere and then triethoxysilane (0.92 mL, 5.0 mmol) and titanium (IV) isopropoxide (0.03 mL, 0.1 mmol) were added. The reaction mixture was heated to 60°C for 15 hours. The C₆H₆ was removed in vacuo and the resulting cream colored solid was dissolved in warm (60°C) hexane (3 mL). The hexane solution was cooled to room temperature, and cream colored crystals appeared in the flask. The recrystallization flask was put in an acetone-filled dewar, which was cooled to -78°C overnight. The hexane solution was decanted from the cream colored crystals which were dried in vacuo, and 0.38 g of product was collected, although ¹H NMR showed the presence of a silicon byproduct. The material was dissolved in 3 mL of warm hexane (60 °C) and slowly cooled to room temperature. The hexane was decanted and the product was dried in vacuo to produce 0.24 g (63% yield) of 4-(2-phenylethenyl)morpholine as a cream colored solid.

### Example 17 (Preparation of 4-(2-[2-thienyl]ethenyl) morpholine

4-(2-[2-thienyl]acetyl) morpholine (0.42 g, 2.0 mmol) was dissolved in C₆H₆ (4 mL) and then triethoxysilane (0.92 mL, 5.0 mmol) and titanium (IV) isopropoxide (0.03 mL, 0.1 mmol) were added. The reaction mixture was heated to 60 °C for 15 hours. The C₆H₆ was removed in vacuo and the resulting yellow solid was dissolved in warm (60°C) hexane (4 mL). The hexane solution was cooled to room temperature, and yellow crystals appeared in the flask. The recrystallization flask was put in an acetone-filled dewar, which was cooled to -78°C overnight. The hexane solution was decanted from the Yellow crystals. The crystals were washed with cold hexane (3 mL) and recrystallized from hot (60°C) hexane. The flask containing the crystals was put in an acetone-filled dewar, which was cooled to -78°C. The hexane was decanted and the crystals were washed with 2 portions of cold hexane (1 mL). The crystals were dried in vacuo to give 0.28 g (74% yield) of 4-(2-[2-thienyl]ethenyl) morpholine, as yellow crystals.

### Example 18 (Preparation of N,N-dimethylbenzylamine)

N,N-dimethylbenzamide (0.45 g, 3.0 mmol), triethoxysilane (1.4 mL, 7.5 mmol), titanium (IV) isopropoxide (0.04 mL, 0.15 mmol), and C₆H₆ (0.5 mL) were added to a flask open to the air and capped with a CaSO₄ drying tube. The reaction mixture was heated to 60°C for 16 hours. The solvent was removed in vacuo and the contents were added to a mixture of 1 M NaOH (20mL) and THF (10mL). This mixture was stirred for 3 hours at room temperature and then poured into ethyl ether and washed with 1 M NaOH (5x50mL). The ether extracts were dried over MgSO₄, filtered and the solvent was removed in vacuo to produce 0.30 g of N,N-dimethylbenzylamine as a yellowish oil (74% yield).

### Example 19 (Preparation of N-benzylpyrrole)

N-benzylsuccinimide (0.38 g, 2.0 mmol), triethoxysilane (1.85 mL, 10 mmol), and titanium (IV) isopropoxide (0.06 mL, 0.2 mmol) were dissolved in C₆H₆ (4.0 mL) and the mixture was heated to 60 °C for 16 hours. The solvent was removed in vacuo and the reaction mixture was poured into a mixture of 1 M NaOH (10 mL) and THF (10 mL). This mixture was stirred for 1 hour, poured into ethyl ether (75 mL), and washed with 1 M NaOH (5 x 50 mL). The ether layer was dried over MgSO₄, filtered, and the solvent was removed in vacuo to produce 0.29 g (91 % yield) of N-benzylpyrrole as a yellow oil.

### Example 20 (Preparation of Chrysanthemumyl alcohol)

A dry Schlenk tube under argon was charged with trichlorotitanium (IV) isopropoxide (35 mg, 0.25 mmol) and triethoxysilane (1.38 mL, 7.5 mmol) and heated to 40°C. Ethyl chrysanthemumate (650 µL, 3 mmol) was then added, and the reaction mixture was stirred at 40°C. After 4 days, the reaction was not yet complete. An additional 300 µL of triethoxysilane was added. After an additional 24 hours, standard work-up afforded 429 mg of a yellow oil, a mixture of cis and trans isomers of chrysanthemumyl alcohol.

### Example 21 (Preparation of Decanol)

A dry Schlenk tube under argon was charged with 48 mg (0.15 mmol) of niobium (V) ethoxide. Triethoxysilane (1.4 mL, 7.5 mmol) and ethyl decanoate (696 µL, 3 mmol) were added and the reaction mixture was heated to 50°C. After 3 hours, the reaction was complete, as determined by GLC analysis of an aliquot taken from the reaction mixture. THF (8 mL) and aqueous NaOH (1 N, 15mL) were then added, and the mixture was stirred vigorously for 3.5 hours. The reaction was worked up as follows: The reaction mixture was added to a water/ether mixture (50 mL each) and shaken vigorously. The two layers were separated, and the aqueous layer was extracted with an additional 50 mL of ether. The combined organic layers were then dried over MgSO₄, filtered, and concentrated. Purification by flash chromatography (ether:hexane = 1:1) afforded 370 mg of a clear oil with a flaky precipitate suspension. Filtering the oil through a small plug of Celite® afforded 343 mg (72% yield) of pure decanol (>95% pure by ¹H-NMR analysis).

### Example 22 (Preparation of Chrysanthemumyl alcohol)

A dry Schlenk tube under argon was charged with 48 mg (0.15 mmol) of niobium (V) ethoxide. Triethoxysilane (1.4 mL, 7.5 mmol) and ethyl chrysanthemumate (650 µL, 3 mmol) were added and the reaction mixture was heated to 50°C. After 5 days, the reaction was complete, as determined by GLC analysis of an aliquot taken from the reaction mixture. THF (8 mL) and aqueous NaOH (1 N, 15 mL) were then added, and the mixture was stirred vigorously for 4 hours. The reaction was worked up as in Example 25. Purification by flash chromatography (ether:hexane = 1:1) afforded 320 mg (69% yield) of a yellow oil, a mixture of cis and trans isomers of chrysanthemumyl alcohol (>95% pure by GC analysis).

### Example 23 (Preparation of Decanol)

A dry Schlenk tube under argon was charged with 48 mg (0.15 mmol) of neodymium (III) isopropoxide. Triethoxysilane (1.4 mL, 7.5 mmol) and ethyl decanoate (696 µL, 3 mmol) were added and the reaction mixture was heated to 60°C. After 7 hours exposure of the reaction mixture to air caused a flame, presumably due to SiH₄ gas evolution. After 29 hours, THF (8 mL) and aqueous NaOH (1 N, 15 mL) were added, and the mixture was stirred vigorously for 2.5 hours. The reaction was worked up as in Example 25. Purification by flash chromatography (ether:hexane = 3:7) afforded 114 mg (24% yield) of decanol and 263 mg (44% yield) of recovered starting material (both >95% pure by ¹H-NMR analysis).

### Example 24 (Preparation of Decanol)

A dry Schlenk tube under argon was charged with 51 mg (0.15 mmol) of dysprosium (III) isopropoxide. Triethoxysilane (1.4 mL, 7.5 mmol) and ethyl decanoate (696 µL, 3 mmol) were added and the reaction mixture was heated to 60°C. After 29 hours, GLC analysis of an aliquot taken from the reaction mixture showed 23% conversion. The reaction mixture was then heated to 70°C. After an additional 3 days, THF (8 mL) and aqueous NaOH (1 N, 15 mL) were added, and the mixture was stirred vigorously for 3 hours. The reaction was worked up as in Example 25. Purification by flash chromatography (ether:hexane = 3:7) afforded 224 mg (47% yield) of decanol (>95% pure by ¹H-NMR analysis) and 40 mg of recovered starting material (80% pure by GC, 5.5% yield).

### Example 25 (Preparation of 4-phenyl-2-butanol)

A dry Schlenk tube under argon was charged with 48 mg (0.15 mmol) of neodymium (III) isopropoxide. Triethoxysilane (1.4 mL, 7.5 mmol) was added, and the reaction mixture was heated to 60°C. After 0.5 hours, 4-phenyl-2-butanone (450 µL, 3 mmol) was added. After 24 hours, the reaction was complete, as determined by GLC analysis of an aliquot taken from the reaction mixture. THF (8 mL) and aqueous NaOH (1 N, 15 mL) were added, and the mixture was stirred vigorously for 12 hours. The mixture was added to water and ether (50 mL of each), shaken vigorously, and separated. The aqueous layer was then extracted with an additional 50 mL of ether, and the combined organic layers were dried over MgSO₄. After removal of the drying agent by filtration and concentration of the solution by rotary evaporation, the crude material was purified by flash chromatography (ether:hexane = 3:7) to afford 245 mg (54% yield) of 4-phenyl-2-butanol (>95% pure by ¹H-NMR analysis).

### Example 26 (Preparation of 4-phenyl-2-butanol)

A dry Schlenk tube under argon was charged with 51 mg (0.15 mmol) of dysprosium (III) isopropoxide. Triethoxysilane (1.4 mL, 7.5 mmol) was added, and the reaction mixture was heated to 60°C. After 0.5 hours, 4-phenyl-2-butanone (450 µL, 3 mmol) was added. After 24 hours, the reaction was complete, as determined by GLC analysis of a aliquot taken from the reaction mixture. THF (8 mL) and aqueous NaOH (1 N, 15 mL) were added, and the mixture was stirred vigorously for 12 hours. After work-up as in Example 25, the crude material was purified by flash chromatography (ether:hexane = 3:7) to afford 234 mg (52% yield) of 4-phenyl-2-butanol (>95% pure by ¹H-NMR analysis).

### Example 27 (Preparation of 4-phenyl-2-butanol)

A dry Schlenk tube under argon was charged with 37 mg (0.15 mmol) of yttrium (III) isopropoxide (Y₅O(iPrO)₁₃). Triethoxysilane (1.4 mL, 7.5 mmol) was added, and the reaction mixture was heated to 60°C. After 0.5 hours, 4-phenyl-2-butanone (450 µL, 3 mmol) was added. After 24 hours, the reaction was complete, as determined by GLC analysis of an aliquot taken from the reaction mixture. THF (8 mL) and aqueous NaOH (1 N, 15 mL) were added, and the mixture was stirred vigorously for 12 hours. After work-up as in Example 25, the crude material was purified by flash chromatography (ether:hexane = 3:7) to afford 235 mg of 4-phenyl-2-butanol.

With respect to the above examples, it is noted that the reactions were run under an atmosphere of nitrogen, argon or hydrogen, except where otherwise noted. Further, the tetrahydrofuran, diethyl ether and benzene used as reaction solvents in the examples were distilled under argon from sodium/benzophenone ketyl before use. Hexane was deolefinated by stirring over H₂SO₄ and stored over CaH₂ before distillation from sodium/benzophenone ketyl under argon. The titanocene dichloride was purchased from Boulder Scientific Inc. of Mead Colorado, and was used without further purification. The (R,R)-Ethylene-1,2-bis(η⁵-4,5,6,7-tetrahydroindenyl) titanium (R)-1,1'-binaphth-2,2'-diolate catalyst was prepared according to the process of Wild et al., J. Orqanomet. Chem., 1982, 232, 233.

The above examples are intended to be illustrative of the invention and should not be read to limit the invention to the specific reduction reactions provided in the examples. One skilled in the art will readily appreciate that the invention is applicable to a variety of reduction reactions in which the substrate is an ester, a lactone, a ketone, an amide, or an imine, and that a variety of catalysts may be used in these reduction reactions. While the examples demonstrating the reduction of esters to alcohols were, where noted, conducted in air, it is understood that such reactions may be conducted in an inert atmosphere, such as argon or nitrogen, as well.

## Claims

1. A process for catalytically reducing organic carbonyl compounds, comprising the steps of:
providing a catalytic amount of an active species of a catalyst selected from the group consisting of a complex of a group 4, 5 or 6 metal which is capable of being converted to a complex in less than its maximum oxidation state, a complex of a group 4, 5 or 6 metal in less than its maximum oxidation state, a group 4, 5 or 6 metal hydride complex, and a group 4, 5 or 6 complex which is capable of being converted into a metal hydride complex;
adding a stoichiometric amount of a silane compound able to contribute a hydride ion during a reduction reaction;
reacting an organic carbonyl substrate selected from the group consisting of acyclic esters, cyclic esters, and amides with the silane compound in the presence of said catalyst at a temperature between about room temperature and 60°C; and
recovering and purifying the reaction product.

2. The process of claim 1 wherein said catalyst is a titanium-containing catalyst selected from the group consisting of L(L')(L")Ti, L(L')(L")Ti-X, L(L')(L")(L''')Ti, L(L')Ti-X, L(L')Ti-X₂ and L(L')Ti-H, where X is a halogen, and where L, L', L" and L"' can be -OR, -SR, -NR(R'), R, Si(R)(R')(R"), and P(R)(R')(R"), or a cyclopentadienyl group of the structure 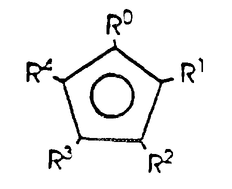 where R, R' and R" can be hydrogen, alkyl, aryl, hydride or silyl groups, and R⁰, R¹, R, R³ and R⁴ may be hydrogen, alkyl, aryl, trialkylsilyl, triarylsilyl, (dialkyl)arylsilyl, or (diaryl)alkylsilyl groups in any combination.

3. The process of claim 1 wherein the silane compound is selected from the group consisting of silane, diphenylsilane, phenylsilane, diethylsilane, dimethylsilane, triethoxysilane, and poly(methylhydrosiloxane).

4. The process of claim 3 wherein following the step of reacting the substrate with the silane compound in the presence of the catalysts, the process further comprises the step of cleaving silicon from the resulting reaction product.

5. A catalytic asymmetric reduction process, comprising the steps of:
providing a catalytic amount of an active species of an enantiomerically enriched chiral catalyst selected from the group consisting of M(L)(L')(L"), M(L)(L')(L")(L"'), M(L)(L')(L")(L''')(L^{iv}), and M(L)(L')(L")(L''')(L^{iv})(L^{v}), where M is a group 3, 4, 5, or 6 metal, a lanthanide or an actinide, and L, L', L", L"', L^{iV}, L^{V}, independently, is some combination of H, alkyl, aryl , Si(R)(R')(R"), P(R)(R')(R"), halogen, -OR, -SR, -NR(R'), or a cyclopentadienyl group having the formula 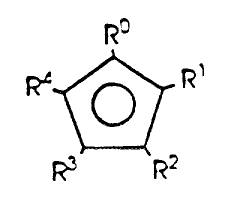 where R, R' and R" is H, alkyl, aryl, or silyl and may be different or the same, and where R⁰, R¹, R, R³, and R⁴ may be hydrogen, alkyl, aryl, trialkylsilyl, triarylsilyl, (dialkyl)arylsilyl, or (diaryl)alkylsilyl groups in any combination;
adding a silane compound to the catalyst;
reacting a substrate, selected from the group consisting of imines, oximes, hydrazones, oxime O-alkyl ethers, oxime O-aryl ethers, N,N-dialkylhydrazones, N,N-diarylhydrazones, and N-alkyl-N-arylhydrazones, in the presence of said catalyst and the silane compound; and
recovering and purifying an amine reaction product having a high level of enantiomeric purity.

6. The process of claim 5 wherein the catalyst is an enantiomerically enriched chiral bis(cyclopentadienyl) titanium complex.

7. The process of claim 6 wherein the catalyst is an enantiomerically enriched chiral complex selected from the group consisting of chiral bis(cyclopentadienyl) titanium monohalide complexes, chiral bis(cyclopentadienyl) titanium monoalkoxide complexes, chiral bis(cyclopentadienyl) titanium monoaryloxide complexes, chiral bis(cyclopentadienyl) titanium dihalide complexes, chiral bis(cyclopentadienyl)titanium dialkoxide complexes, chiral bis(cyclopentadienyl) titanium diaryloxide complexes, and chiral bis(cyclopentadienyl) titanium aryloxide alkoxide complexes.

8. The process of claim 7 wherein the catalyst is enantiomerically enriched with a compound selected from the group consisting of(R,R)-Ethylene-1,2-bis (η⁵-4, 5, 6, 7-tetrahydroindenyl) titanium (R)-1,1'-binaphth-2,2'-diolate; or (S,S)-Ethylene-1,2-bis (η⁵-4, 5, 6, 7-tetrahydroindenyl) titanium (S)-1,1'-binaphth-2,2'-diolate.

9. The process of claim 5 wherein the silane compound is selected from the group consisting of silane, diphenylsilane, phenylsilane, diethylsilane, dimethylsilane, triethoxysilane, trimethoxysilane and poly(methylhydrosiloxane).

10. The process of claim 5 wherein an enantiomerically enriched quantity of the catalyst constitutes in excess of 80% of one enantiomer of the catalyst.

11. The process of claim 5 wherein the silane compound is added in a stoichiometric quantity relative to the substrate.

12. The process of claim 11 wherein following the step of reacting the substrate in the presence of the catalyst and the silane compound, the process further includes the step of cleaving silicon from the resulting reaction product.

13. The process of claim 5 wherein the step of reacting the substrate in the presence of the catalyst and the silane compound is conducted in a hydrogen atmosphere, where the hydrogen acts as a reducing agent.

14. A catalytic asymmetric reduction process, comprising the steps of:
providing a mixture of (i) a catalytic amount of an active species of a catalyst selected from the group consisting of M(L)(L')(L"), M(L)(L')(L")(L'''), M(L)(L')(L")(L''')(L^{iv}) and M(L)(L')(L'')(L''')(L^{iv})(L^{v}), where M is a group 3, 4, 5, or 6 metal, a lanthanide or an actinide, and L, L', L", L"', L^{iv}, L^{v}, independently, is some combination of H, alkyl, aryl, Si(R)(R')jR"), P(R)(R')(R"), halogen, -OR, -SR, or -NR(R'), or a cyclopentadienyl group having the formula 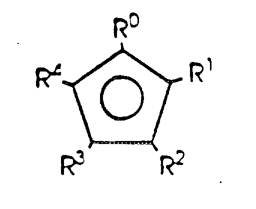 where R, R' and R" may be H, an alkyl, aryl, or silyl group and may be different or the same, and where R⁰, R¹, R, R³, and R⁴ may be hydrogen, alkyl, aryl, trialkylsilyl, triarylsilyl, (dialkyl)arylsilyl, or (diaryl)alkylsilyl groups in any combination; (ii) an enantiomerically enriched chiral additive selected from the group consisting of amines, diamines, alcohols, diols, organic acids, organic diacids, thiols and phosphines; and (iii) a silane compound able to supply a hydride ion during the reduction reaction;
reacting a substrate, selected from the group consisting of, imines, oximes, hydrazones, oxime O-alkyl ethers, oxime O-aryl ethers, N,N-dialkylhydrazones, N,N-diarylhydrazones, and N-alkyl-N-arylhydrazones in the presence of the mixture; and
recovering and purifying an amine reaction product enriched in one enantiomer.

15. The process of claim 14 wherein the catalyst is selected from the group consisting of metal alkoxides and metal aryloxides.

16. The process of claim 15 wherein the catalyst is selected from the group consisting of titanium (IV) isopropoxide, titanium (IV) ethoxide, titanium (IV) propoxide, titanium (IV) methoxide, trichlorotitanium (IV) isopropoxide, and titanium (IV) butoxide.

17. The process of claim 14 wherein the silane compound is selected from the group consisting of silane, diphenylsilane, phenylsilane, diethylsilane, dimethylsilane, triethoxysilane, trimethoxysilane and poly(methylhydrosiloxane).

18. The process of claim 14 wherein the chiral additive is present in an amount ranging between about 0.1 and 100 mole %, relative to the amount of substrate.

19. The process of claim 18 wherein the chiral additive is selected from the group consisting of (1R, 2R)-diaminocyclohexane; (1S, 2S)-diaminocyclohexane; (R)-1,1'-Bi-2-naphthol, (S)- 1,1'-Bi-2-naphthol; (1R, 2S)-ephedrine; (1S, 2R)-ephedrine; and 1,1,4,4-tetraphenyl-2,3-O-isopropylidene-D-threitol.

20. The process of claim 19 wherein following the step of reacting the substrate in the presence of the mixture, the process further includes the step of cleaving silicon from the resulting reaction product.

21. A process for catalytically reducing organic carbonyl compounds, comprising the steps of:
providing a stoichiometric quantity of a silane reducing agent able to contribute a hydride ion during the reduction reaction, a catalytic amount of a catalyst selected from the group consisting of M(L)(L')(L"), M(L)(L')(L")(L'''), M(L)(L')(L")(L''')(L^{iv}), and M(L)(L')(L")(L"') (L^{iv})(L^{V}), where M is a group 3, 4, 5 or 6 metal, a lanthanide or an actinide and L, L', L", L"', L^{iv}, L^{v}, independently, are some combination of H, alkyl, aryl, silyl, P(R)(R')(R"), halogen, -OR, -SR, or -NR(R'), where R, R', and R" are H, alkyl, aryl, or silyl and may be different or the same, and a stoichiometric quantity of an organic carbonyl substrate selected from the group consisting of esters, lactones, amides and imides;
reacting the organic carbonyl substrate, in the presence of the catalyst and the silane compound at a temperature between 25° and 80°; and
recovering and purifying the reaction product.

22. The process of claim 21 wherein the catalyst is a metal alkoxide or a metal aryloxide complex.

23. The process of claim 22 wherein said catalyst is selected from the group consisting of titanium (IV) isopropoxide, titanium (IV) ethoxide, trichlorotitanium (IV) isopropoxide, niobium (V) ethoxide, neodymium (III) isopropoxide, dysprosium (III) isopropoxide, and yttrium (III) isopropoxide.

24. The process of claim 21 wherein the silane reducing agent is selected from the group consisting of silane, diphenylsilane, phenylsilane, diethylsilane, dimethylsilane, triethoxysilane, trimethoxysilane, and poly(methylhydrosiloxane).

25. A process for the catalytic asymmetric reduction of ketones, comprising the steps of:
providing a mixture of (i) a catalytic amount of an active species of a catalyst selected from the group consisting of M(L)(L')(L"), M(L)(L')(L")(L'''), M(L)(L')(L'')(L''')(L^{iv}), and M(L)(L')(L'')(L''')(L^{iv})(L^{v}), where M is a group 3, 4, 5 or 6 metal, a lanthanide or an actinide, and L, L', L", L"', L^{iv}, L^{v}, independently, are some combination of H, alkyl, aryl, a silyl, P(R)(R')(R"), halogen, -OR, -SR, or -NR(R'), where R, R', and R" are H, alkyl, aryl, silyl and may be different or the same, (ii) a stoichiometric amount of a silane compound able to supply a hydride ion during the reduction reaction, and (iii) an enantiomerically enriched chiral additive selected from the group consisting of amines, diamines, alcohols, diols, organic acids, organic diacids, thiols, and phosphines;
reacting a ketone substrate in the presence of the mixture; and
recovering and purifying an alcohol reaction product enriched in one enantiomer.

26. The process of claim 25 wherein the catalyst is selected from the group consisting of metal alkoxides and metal aryloxides.

27. The process of claim 26 wherein the catalyst is selected from the group consisting of titanium (IV) isopropoxide, trichlorotitanium (IV) isopropoxide, titanium (IV) ethoxide, titanium (IV) methoxide, and titanium (IV) butoxide.

28. The process of claim 25 wherein the silane compound is selected from the group consisting of silane, diphenylsilane, phenylsilane, diethylsilane, dimethylsilane and triethoxysilane, trimethoxysilane and poly(methylhydrosiloxane).

29. The process of claim 25 wherein the amount of chiral additive is present in an amount ranging between about 0.1 and 100 mole percent, relative to the amount of substrate.

30. The process of claim 29 wherein the chiral additive is selected from the group consisting of (1R, 2R)-diaminocyclohexane; (1S, 2S)-diaminocyclohexane; (R)-1, 1'-Bi-2-naphthol, (S)-1,1'-Bi-2-naphthol; (1R, 2S)-ephedrine; (1S, 2R)-ephedrine; and 1,1,4,4-tetraphenyl-2, 3-O-isopropylidene-D-threitol.

## Patentansprüche

1. Verfahren, um organische Carbonylverbindungen katalytisch zu reduzieren, das die folgenden Schritte umfasst:
eine katalytische Menge einer aktiven Art eines Katalysators wird geliefert, der von der Gruppe ausgewählt ist, die aus einem Komplex aus einem Metall der Gruppe 4, 5 oder 6 besteht, der in einen Komplex umgewandelt werden kann, der kleiner als sein maximaler Oxidationszustand ist, einem Komplex aus einem Metall der Gruppe 4, 5 oder 6, der kleiner als sein maximaler Oxidationszustand ist, einem Metallhydridkomplex der Gruppe 4, 5 oder 6, und einem Komplex der Gruppe 4, 5, oder 6, der in einen Metallhydridkomplex umgewandet werden kann;
eine stöchiometrische Menge einer Silanverbindung wird zugegeben, die während einer Reduktionsreaktion ein Hydridion beitragen kann;
ein organisches Carbonylsubstrat, das von der Gruppe ausgewählt ist, die aus azyclischen Estern, zyklischen Estern, und Amiden besteht, wird mit der Silanverbindung in Gegenwart des Katalysators bei einer Temperatur zwischen ungefähr Zimmertemperatur und 60°C reagiert; und
das Reaktionsprodukt wird regeneriert und gereinigt.

2. Verfahren nach Anspruch 1, in dem der Katalysator ein Titan enthaltender Katalysator ist, der von der Gruppe ausgewählt ist, die aus L(L')(L'')Ti, L(L')(L'')Ti-X, L(L')(L'')(L''')Ti, L(L')Ti-X₂ und L(L')Ti-H besteht, und worin L,L',L'' und L''' -OR, -SR, -NR(R'), R, Si(R)(R')(R''), und P(R)(R')(R''), oder eine Cyclopentadienalgruppe mit der Struktur 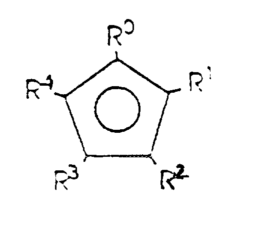 sein können, worin R, R', R" Wasserstoff-, Alkyl-, Aryl-, Hydrid- oder Silylgruppen sein können, und R⁰, R¹, R, R³ und R⁴ Wasserstoff-, Alkyl-, Aryl-, Trialkylsilyl-, Triarylsilyl-, (Dialkyl)arylsilyl-, oder (Diaryl)alkylsilylgruppen in irgendeiner Kombination sein können.

3. Verfahren nach Anspruch 1, in dem die Silanverbindung von der Gruppe ausgewählt ist, die aus Silan, Diphenylsilan, Phenylsilan, Diethylsilan, Dimethylsilan, Triethoxysilan, und Poly(methylhydrosiloxan) besteht.

4. Verfahren nach Anspruch 3, in dem das Verfahren nach dem Schritt der Reaktion des Substrats mit der Silanverbindung in Gegenwart der Katalysatoren weiterhin den Schritt umfasst, Silicium von dem sich ergebenden Reaktionsprodukt abzutrennen.

5. Katalytisches asymmetrisches Reduktionsverfahren, das die folgenden Schritte umfasst:
eine katalytische Menge einer aktiven Art von einem enantiomerisch angereicherten Chiralkatalysator wird geliefert, der von der Gruppe ausgewählt ist, die aus M(L)(L')(L''), M(L)(L')(L'')(L'''), M(L)(L')(L'')(L''')(L^{iv}), und M(L)(L')(L'')(L''')(L^{iv})(L^{v}) besteht, worin M ein Metall der Gruppe 3, 4, 5, oder 6 ist, ein Lanthanid oder ein Actinid, und L, L', L'', L''', L^{iv}, L^{v}, unabhängig, eine Kombination von H, Alkyl, Aryl, Si(R)(R')(R''), P(R)(R')(R''), Halogen, -OR, -SR, -NR(R'), oder eine Cyclopentadienylgruppe mit der Formel 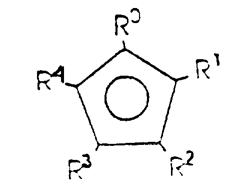 ist, worin R, R'und R'' H, Alkyl, Aryl oder Silyl ist, und verschieden oder gleich sein können, und worin R⁰, R¹, R, R³ und R⁴ Wasserstoff-, Alkyl-, Aryl-, Trialkylsilyl-, Triarylsilyl-, (Dialkyl)arylsilyl-, oder (Diaryl)alkylsilylgruppen in irgendeiner Kombination sein können;
dem Katalysator wird eine Silanverbindung zugegeben;
ein Substrat, das von der Gruppe ausgewählt ist, die aus Iminen, Oximen, Hydrazonen, Oxim-O-Alkylethern, Oxim-O-Arylethern, N,N-Dialkylhydrazonen, N,N-Diarylhydrazonen, und N,N-Alkyl-N-Arylhydrazonen besteht, wird in Gegenwart des Katalysators und der Silanverbindung reagiert; und
ein Aminreaktionsprodukt mit einem hohen Stand enantiomerischer Reinheit wird regeneriert und gereinigt.

6. Verfahren nach Anspruch 5, in dem der Katalysator ein enantiomerisch angereicherter Chiralbis(cyclopentadienyl)titankomplex ist.

7. Verfahren nach Anspruch 6, in dem der Katalysator ein enantiomerisch angereicherter Chiralkomplex ist, der von der Gruppe ausgewählt ist, die aus Chiralbis(cyclopentadienyl)titanmonohalidkomplexen, Chiralbis(cyclopentadienyl)titanmonoalkoxidkomplexen, Chiralbis(cyclopentadienyl)titanmonoaryloxidkomplexen, Chiralbis(cyclopentadienyl)titandihalidkomplexen, Chiralbis(cyclopentadienyl)titandialkoxidkomplexen, Chiralbis(cyclopentadienyl)titandiaryloxidkomplexen, und Chiralbis(cyclopentadienyl)titanaryloxidalkoxidkomplexen besteht.

8. Verfahren nach Anspruch 7, in dem der Katalysator mit einer Verbindung enantiomerisch angereichert ist, die von der Gruppe ausgewählt ist, die aus (R,R)-Ethylen-1,2-bis( ⁵-4, 5, 6, 7-tetrahydroindenyl)titan(R)-1,1'-binaphth-2,2'-diolat; oder (S,S)-Ethylen-1,2-bis( ⁵-4, 5, 6, 7-tetrahydroindenyl)titan(S)-1,1'-binaphth-2,2'-diolat besteht.

9. Verfahren nach Anspruch 5, in dem die Silanverbindung von der Gruppe ausgewählt ist, die aus Silan, Diphenylsilan, Phenylsilan, Diethylsilan, Dimethylsilan, Triethoxysilan, Trimethoxysilan und Poly(methylhydrosiloxan) besteht.

10. Verfahren nach Anspruch 5, in der eine enantiomerisch angereicherte Menge des Katalysators Über 80% eines Enantiomers des Kalysators enthält.

11. Verfahren nach Anspruch 5, in dem die Silanverbindung in einer stöchiometrischen Menge relativ zu dem Substrat zugegeben wird.

12. Verfahren nach Anspruch 11, in dem das Verfahren nach dem Schritt der Reaktion des Substrats in Gegenwart des Katalysators und der Silanverbindung weiterhin den Schritt einschließt, Silicium von dem sich ergebenden Reaktionsprodukt abzutrennen.

13. Verfahren nach Anspruch 5, in dem der Schritt der Reaktion des Substrats in Gegenwart des Katalysators und der Silanverbindung in einer Wasserstoffatmosphäre durchgeführt wird, worin der Wasserstoff als ein Reduktionsmittel wirkt.

14. Katalytisches asymmetrisches Reduktionsverfahren, das die folgenden Schritte umfasst:
eine Mischung von (i) einer katalytischen Menge einer aktiven Art eines Katalysators, der von der Gruppe ausgewählt ist, die aus M(L)(L')(L''), M(L)(L')(L'')(L'''), M(L)(L')(L'')(L''')(L^{iv}), und M(L)(L')(L'')(L''')(L^{iv})(L^{v}) besteht, worin M ein Metall der Gruppe 3, 4, 5, oder 6 ist, ein Lanthanid oder ein Actinid, und L, L', L'', L''', L^{iv}, L^{v}, unabhängig, eine Kombination von H, Alkyl, Aryl, Si(R)(R')(R''), P(R)(R')(R''), Halogen, -OR, -SR, -NR(R'), oder eine Cyclopentadienylgruppe mit der Formel 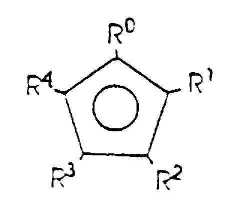 ist, worin R, R'und R'' H, eine Alkyl-, Aryl- oder Silylgruppe sein können, und verschieden oder gleich sein können, und worin R⁰, R¹, R, R³ und R⁴ Wasserstoff-, Alkyl-, Aryl-, Trialkylsilyl-, Triarylsilyl-, (Dialkyl)arylsilyl-, oder (Diaryl)alkylsilylgruppen in irgendeiner Kombination sein können; (ii) einer enantiomerisch angereicherten Chiralzugabe, die von der Gruppe ausgewählt ist, die aus Aminen, Diaminen, Alkoholen, Diolen, organischen Säuren, organischen zweibasigen Säuren, Thiolen und Phosphinen besteht; und (iii) einer Silanverbindung, die während der Reduktionsreaktion ein Hydridion zubringen kann, wird geliefert;
ein Substrat, das von der Gruppe ausgewählt ist, die aus Iminen, Oximen, Hydrazonen, Oxim-O-Arylethern, N,N-Dialkylhydrazonen, und N-Alkyl-N-arylhydrazonen besteht, wird in Gegenwart der Mischung reagiert; und
ein Aminreaktionsprodukt mit einem hohen Stand enantiomerischer Reinheit wird wiedergewonnen und gereinigt.

15. Verfahren nach Anspruch 14, in dem der Katalysator von der Gruppe ausgewählt ist, die aus Metallalkoxiden und Metallaryloxiden besteht.

16. Verfahren nach Anspruch 15, in dem der Katalysator von der Gruppe ausgewählt ist, die aus Titan(IV)isopropoxid, Titan(IV)ethoxid, Titan(IV)propoxid, Titan(IV)methoxid, Trichlorotitan(IV)isopropoxid, und Titan(IV)butoxid besteht.

17. Verfahren nach Anspruch 14, in dem die Silanverbindung von der Gruppe ausgewählt ist, die aus Silan, Diphenylsilan, Phenylsilan, Diethylsilan, Dimethylsilan, Triethoxysilan, Trimethoxysilan und Poly(methylhydrosiloxan) besteht.

18. Verfahren nach Anspruch 14, in dem die Chiralzugabe in einer Menge vorhanden ist, die von 0,1 bis 100 Mol% relativ zur Menge des Substrats reicht.

19. Verfahren nach Anspruch 18, in dem die Chiralzugabe von der Gruppe ausgewählt ist, die aus (1R, 2R)-Diaminocyclohexan; (1S, 2S)-Diaminocyclohexan; (R)-1,1'-Bi-2-naphthol, (S)-1,1'-Bi-2-naphthol; (1R, 2S)-Ephedrin; (1S, 2R)-Ephedrin; und 1,1,4,4-Tetraphenyl-2,3-O-isopropyliden-D-threitol besteht.

20. Verfahren nach Anspruch 19, in dem das Verfahren nach dem Schritt der Reaktion des Substrats in Gegenwart der Mischung weiterhin den Schritt einschließt, Silicium von dem sich ergebenden Reaktionsprodukt abzutrennen.

21. Verfahren zum katalytischen Reduzieren von organischen Carbonylverbindungen, das die folgenden Schritte umfasst:
eine stöchiometrische Menge eines Silan reduzierenden Mittels wird geliefert, die während der Reduktionsreaktion ein Hydridion beitragen kann, eine katalytische Menge eines Katalysators, der von der Gruppe ausgewählt ist, die aus M(L)(L')(L''), M(L)(L')(L'')(L'''), M(L)(L')(L'')(L''')(L^{iv}), und M(L)(L')(L')(L'')(L^{iv})(L^{v}) besteht, worin M ein Metall der Gruppe 3, 4, 5, oder 6 ist, ein Lanthanid oder ein Actinid, und L, L', L'', L''', L^{iv}, L^{v}, unabhängig, eine Kombination von H, Alkyl, Aryl, Silyl, P(R)(R')(R''), Halogen, -OR, -SR, -NR(R') sind, worin R, R'und R'' H, Alkyl, Aryl oder Silyl sind, und verschieden oder gleich sein können, und eine stöchiometrische Menge eines organischen Carbonylsubstrats, das von der Gruppe ausgewählt ist, die aus Estern, Lactonen, Amiden und Imiden besteht;
das organische Carbonylsubstrat wird in Gegenwart des Katalysators und der Silanverbindung bei einer Temperatur zwischen 25°C und 80°C reagiert; und
das Reaktionsprodukt wird regeneriert und gereinigt.

22. Verfahren nach Anspruch 21, in dem der Katalysator ein Metallalkoxid- oder ein Metallaryloxidkomplex ist.

23. Verfahren nach Anspruch 22, in dem der Katalysator von der Gruppe ausgewählt ist, die aus Titan(IV)isopropoxid, Titan(IV)ethoxid, Trichlorotitan(IV)isopropoxid, Niobium(V)ethoxid, Neodymium(III)isopropoxid, Dysprosium(III)isopropoxid, und Yttrium(III)isopropoxid besteht.

24. Verfahren nach Anspruch 21, in dem das Silanreduktionsmittel von der Gruppe ausgewählt ist, die aus Silan, Diphenylsilan, Phenylsilan, Diethylsilan, Dimethylsilan, Triethoxysilan, Trimethoxysilan, und Poly(methylhydrosiloxan) besteht.

25. Verfahren zur katalytiscyhen asymmetrischen Reduktion von Ketonen, das die folgenden Schritte umfasst:
eine Mischung von (i) einer katalytischen Menge einer aktiven Art eines Katalysators, der von der Gruppe ausgewählt ist, die aus M(L)(L')(L''), M(L)(L')(L'')(L'''), M(L)(L')(L'')(L''')(L^{iv}), und M(L)(L')(L'')(L''')(L^{iv})(L^{v}) besteht, worin M ein Metall der Gruppe 3, 4, 5, oder 6 ist, ein Lanthanid oder ein Actinid, und L, L', L'', L''', L^{iv}, L^{v} unabhängig eine Kombination von H, Alkyl, Aryl, Silyl, P(R)(R')(R''), Halogen, -OR, -SR, oder -NR(R') sind, worin R, R'und R'' H, Alkyl, Aryl oder Silyl sind, und verschieden oder gleich sein können, und (ii) einer stöchiometrischen Menge einer Silanverbindung, die während der Reduktionsreaktion ein Hydridion zubringen kann, und (iii) einer enantiomerisch angereicherten Chiralzugabe, die von der Gruppe ausgewählt ist, die aus Aminen, Diaminen, Alkoholen, Diolen, organischen Säuren, organischen Disäuren, Thiolen, und Phosphinen besteht, wird geliefert;
ein Ketonsubstrat wird in Gegenwart der Mischung reagiert; und ein Alkoholreaktionsprodukt, das in einem Enantiomer angereichert ist, wird regeneriert und gereinigt.

26. Verfahren nach Anspruch 25, in dem der Katalysator von der Gruppe ausgewählt ist, die aus Metalloxiden und Metallaryloxiden besteht.

27. Verfahren nach Anspruch 26, in dem der Katalysator von der Gruppe ausgewählt ist, die aus Titan(IV)isopropoxid, Trichlorotitan(IV)isopropoxid, Titan(IV)ethoxid, Titan(IV)methoxid, und Titan(IV)butoxid besteht.

28. Verfahren nach Anspruch 25, in dem die Silanverbindung von der Gruppe ausgewählt ist, die aus Silan, Diphenylsilan, Phenylsilan, Diethylsilan, Dimethylsilan und Triethoxysilan, Trimethoxysilan und Poly(methylhydrosiloxan) besteht.

29. Verfahren nach Anspruch 25, in dem die Menge der Chiralzugabe in einer Menge vorhanden ist, die zwischen ungefähr 0,1 und 100 Molprozent relativ zur Menge des Substrats liegt.

30. Verfahren nach Anspruch 29, in dem die Chiralzugabe von der Gruppe ausgewählt ist, die aus (1R, 2R)-Diaminocyclohexan; (1S, 2S)-Diaminocyclohexan; (R)-1, 1'-Bi-2-naphthol, (S)-1,1'-Bi-2-naphthol; (1R, 2S)-Ephedrin; (1S, 2R)-Ephedrin; und 1,1,4,4-Tetraphenyl-2, 3-O-isopropyliden-D-threitol besteht.

## Revendications

1. Procédé de réduction catalytique de composés organiques carbonyliques, comportant les phases suivantes:
l'apport d'un montant catalytique de catalyseur de classe active sélectionné à partir du groupe consistant d'un complexe de groupe métallique 4, 5 ou 6 susceptible d'être transformé en complexe d'état inférieur à son état d'oxydation maximum, un complexe de groupe métallique 4, 5 ou 6 inférieur à son état d'oxydation maximum, un complexe de groupe 4, 5 ou 6 d'hydrure métallique, et un complexe de groupe 4, 5 ou 6 susceptible d'être transformé en complexe d'hydrure métallique;
l'ajout d'un montant stoechiométrique de composé de silane apte à apporter un ion d'hydrure lors de la réaction de réduction;
la réaction d'un substrat organique carbonylique sélectionné à partir du groupe consistant d'esters acycliques, d'esters cycliques et d'amides avec le composé de silane en présence dudit catalyseur à une température entre approximativement la température ambiante et 60°C; et
la récupération et l'épuration du produit de réaction.

2. Le procédé à la revendication 1 dont ledit catalyseur est à teneur de titane et sélectionné partir du groupe qui comporte L(L')(L")Ti,L(L')(L")Ti-X,L(L')(L")(L''')Ti,L(L')Ti-X,L(L')T1-X₂, et L(L')Ti-H, dont X représente un halogène, et dont L,L',L'' et (L''') peuvent éventuellement représenter -OR,-NR(R'), R, Si(R)(R'') et P(R)(R')(R'') ou un groupe cyclopentadiényle ayant la structure 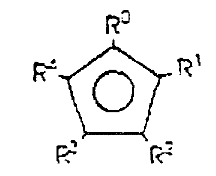 dont R, R' et R" représentent éventuellement l'hydrogène, des groupes alcoyle, aryle ou sylile, et dont R⁰, R¹, R, R³ et R⁴ peuvent représenter toute combinaison quelconque des groupes hydrogène, alcoyle, aryle, trialcoyle silyle, triaryle silyle, (dialcoyle) aryle silyle, ou (diaryle) alcoyle silyle.

3. Le procédé à la revendication 1 dont le composé de silane est sélectionné à partir du groupe consistant de silane, diphényle silane, phényle silane, diéthyle silane, diméthyle silane, triétoxy silane, et poly(méthyle hydrosiloxane).

4. Le procédé à la revendication 3 selon lequel suite à la phase de réaction du substrat avec le composé de silane en présence de catalyseurs, le procédé prévoit en outre la phase de dédoublement de silicium du produit résultant de réaction.

5. Procédé de réduction catalytique asymétrique comportant les phases suivantes:
apport d'un montant catalytique de catalyseur enrichi de façon énantiomère sélectionné à partir du groupe consistant de ML(L)(L')(L''), L(L')(L'')(L''')(L^{iv})(L^{v}), dont M est un métal de groupe 3, 4, 5 ou 6, un lanthanide ou un actinide, et dont indépendamment L, L',L'',L''',L^{iv},L^{v}, représentent une combinaison comportant H, alcoyle, aryle, Si(R)(R')(R''), P(R)(R')(R''), halogène, -OR, -SR, -NR(R'), ou un groupe cyclopentadiényle ayant la formule 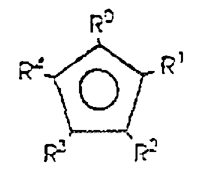 dont R, R' et R" représentent éventuellement H, alcoyle, aryle ou silyle et peuvent être différents ou identiques. et dont R⁰, R¹, R, R³et R⁴ peuvent représenter toute combinaison quelconque des groupes hydrogène, alcoyle, aryle, trialcoyle silyle, triaryle silyle, (dialcoyle) aryle silyle, ou (diaryle) alcoyle silyle.
ajout d'un composé de silane au catalyseur;
la réaction d'un substrat sélectionné à partir du groupe consistant d'imines, oximes, hydrazones, éthers 0-alcoyle oxime, éthers 0-aryle oxime, hydrazones N,N-dialcoyle, hydrazones N,N-diaryle, et hydrazones N-alcoyle-N-aryle, en présence dudit catalyseur et du composé de silane, et
la récupération et l'épuration d'un produit de réaction d'amine comportant une niveau élevé de pureté énantiomère.

6. Le procédé à la revendication 5 selon lequel le catalyseur est un complexe de titane (cyclopentadiényle) bis enrichi de façon énantiomère.

7. Le procédé à la revendication 6 dont le catalyseur est un complexe chiral enrichi de façon énantiamère sélectionné au groupe consistant de complexes monohaloïde de titane (cyclopenta-diényle) chiral bis, de complexes de monoalkoxyde de titane (cyclopentadiényle) chiral bis, de complexes de monoaryle oxyde de titane (cyclopentadiényle) chiral bis, de complexes de dihaloïde de titane (cyclopentadiényle) chiral bis, de complexes de dialkoxyde de titane (cyclopentadiényle) chiral bis, de complexes de diaryle oxyde de titane (cyclopentadiényle) chiral bis, et de complexes alkoxyde diaryle oxyde de titane (cyclopentadiényle) chiral bis.

8. Le procédé à la revendication 7 dont le catalyseur est un complexe de enrichi de façon énantiomère avec un composé sélectionné au groupe consistant de (R,R)éthylène-1,2-bis(η⁵-4,5,6,7 -tétrahydroindényle de titane (R)-1,1'binaphthe-2,2'-diolate; ou (S,S)éthylène-1,2-bis(η⁵-4,5,6,7-tétrahydroindényle de titane (S)-1,1'binaphthe-2,2'-diolate.

9. Le procédé à la revendication 5 dont le composé de silane est sélectionné à partir du groupe consistant de silane, diphényle silane, phényle silane, diéthyle silane, diméthyle silane, triéthoxy silane, triméthoxy silane et poly(méthyle hydrosiloxane).

10. Le procédé à la revendication 5 selon lequel le montant du catalyseur enrichi de façon énantiomère est supérieur à 80% d'un énantiomère du catalyseur.

11. Le procédé à la revendication 5 selon lequel le composé de silane est ajouté à raison d'un montant stoechiométrique par rapport au substrat.

12. Le procédé à la revendication 11 selon lequel suite à la phase de réaction du substrat en présence du catalyseur et du composé de silane, le procédé prévoit en outre la phase de dédoublement du silicium du produit résultant de réaction.

13. Le procédé à la revendication 5 selon lequel la phase de réaction du substrat en présence du catalyseur et du composé de silane est effectuée en atmosphère d'hydrogène, l'hydrogène servant d'agent réducteur.

14. Procédé catalytique asymétrique de réduction comportant les phases;
d'apport de mélange (i) d'un montant catalytique de catalyseur de classe active sélectionné à partir du groupe consistant de ML(L)(L')(L''), L(L')(L'')(L''')(L^{iv}), et de ML(L)(L')(L''),(L''') (L^{iv}) (L^{v}), dont M est un métal de groupe 3, 4, 5 ou 6, un lanthanide ou un actinide, et dont indépendamment L, L',L'',L''', L^{iv},L^{v}, représentent une combinaison comportant H, alcoyle, aryle, Si(R) (R')(R''), P(R)(R')(R''), halogène, -OR, -SR, ou -NR(R'), ou un groupe cyclopentidiényle ayant la formule 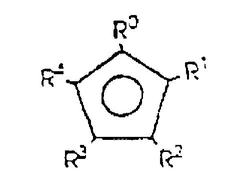 dont R, R' et R'' peuvent être H, un groupe alcoyle, aryle ou silyle et peuvent être différents ou identiques, et dont R⁰, R¹, R, R³ et R⁴ peuvent représenter une combinaison quelconque des groupes hydrogène, alcoyle, aryle, trialcoyle silyle, triaryle silyle, (dialcoyle) aryle silyle, ou (diaryle) alcoyle silyle; ii) un additif enrichi par voie énantiomère, sélectionné au groupe consistant d'amines, de diamines, d'alcools, de diols, d'acides organiques, de diacides organiques, de thiols et de phosphines; et (iii) un composé de silane susceptible d'apporter un ion d'hydrure lors de la réaction de réduction.
la réaction d'un substrat sélectionné à partir du groupe consistant d'imines; d'oximes, d'hydrazones, d'éthers 0-dialcoyle oxime, d'éthers 0-aryle oxime, d'hydrazones N,N-dialcoyle, d'hydrazones N,N-diaryle et d'hydrazones N-alcoyle-N-aryle en présence dudit mélange; et
la récupération et l'épuration d'un produit de réaction d'amine enrichi en énantiomère.

15. Le procédé à la revendication 14 selon lequel le catalyseur est sélectionné à partir du groupe consistant d'alkoxydes métalliques et d'aryloxydes métalliques.

16. Le procédé à la revendication 15 dont le catalyseur est sélectionné à partir du groupe consistant d'isopropoxyde de titane (IV), d'éthoxyde de titane (IV), de propoxyde de titane (IV), de méthoxyde de titane (IV), d'ixopropoxyde de trichlorotitane (IV) et de butoxyde de titane (IV).

17. Le procédé à la revendication 14 dont le composé de silane est sélectionné à partir du groupe consistant de silane, de diphényle silane, de phényle silane, de diéthyle silane, de diméthyle silane, de triéthoxy silane, de triméthoxy silane et de poly(méthylehydrosiloxane).

18. Le procédé à la revendication 14 dont l'additif est présent en un montant allant de 0,1 à 100 moles %, par rapport au montant du substrat.

19. Le procédé à la revendication 18 dont l'additif chiral est sélectionné à partir du groupe consistant de (1R,2R)-diaminocyclo-hexane;(1S,2S)-diaminocyclohexane;(R)-1,1'-Bi-2-naphtole,(S)1,1'-Bi-2-naphtole; (1R,2S)-éphédrine; (1S,2R)-éphédrine; et 1,1,4,4-tétraphényle-2,3-Disopropylidène-D-threitol.

20. Le procédé à la revendication 19 dont suivant la phase de réaction du substrat en présence du mélange, le procédé prévoit en outre la phase de dédoublement du silicium du produit résultant de la réaction.

21. Procédé de réduction catalytique de composés organiques carbonyliques, comportant les phase suivantes.
l'apport d'un montant stoechiométrique d'agent réducteur de silane apte à apporter un ion d'hydrure lors de la réaction de réduction; un montant catalytique de catalyseur sélectionné à partir du groupe formé de ML(L)(L')(L''), L(L')(L'')(L''')(L^{iv}), et ML(L)(L')(L'')(L''')(L^{iv})(L^{v}), dont M est un métal de groupe 3, 4, 5 ou 6, un lanthanide ou un actinide, et dont indépendamment L, L', L'',L''',L^{iv},L^{v}, représentent une combinaison comportant H, alcoyle, aryle, silyle, P(R)(R')(R''), halogène, -OR, -SR, ou -NR(R'), dont R,R', et R''), représentent H, alcoyle, aryle ou silyle et peuvent être différents ou identiques, et un montant stoechiométrique de substrat organique carbonylique sélectionné à partir du groupe formés d'esters, de lactones, d'amides et d'imides;
la réaction du substrat organique carbonylique, en présence du catalyseur et du composé de silane à une température entre 25° et 80°C; et
la récupération et l'épuration du produit de réaction.

22. Le procédé à la revendication 21 selon lequel le catalyseur est un alkoxyde métallique ou un complexe métallique d'aryloxyde.

23. Le procédé à la revendication 22 selon lequel ledit catalyseur est sélectionné à partir du groupe qui consiste d'isopropoxyde de titane (IV), d'éthoxyde de titane (IV), d'isopropoxyde de trichloro-titane (IV), d'éthoxyde de niobium (V), d'isopropoxyde de néodymium (III), d'isopropoxyde de dysprosium III) et isopropoxyde d'yttrium (III).

24. Procédé à la revendication 21 selon lequel le produit de réduction du silane est sélectionné à partir du groupe qui consiste de silane, diphényle silane, phényle silane, diéthyle silane, diméthyle silane, triéthoxy silane, triméthoxy et poly(méthyle hydrosiloxane.

25. Procédé de réduction asymétrique catalytique des cétones, comportant les phases:
d'apport du mélange (i) d'un montant catalytique de catalyseur de classe active sélectionné à partir du groupe consistant de M(L)(L')(L''), M(L)(L')(L'')(L'''), M(L)(L')(L'')(L''')(L^{iv}), et de M(L)(L')(L''),(L''')(L^{iv})(L^{v}), dont M est un métal de groupe 3, 4, 5 ou 6, un lanthanide au actinide, et dont indépendamment L, L',L'', L''',L^{iv},L^{v}, représentent une combinaison comportant H, alcoyle, aryle, silyle, P(R)(R')(R"), halogène, -DR, -SR, ou NR(R') , dont R,R' et R" sont H, alcoyle, aryle, silyle et peut être différent ou identique, (ii) un montant stoechiométrique de composé de silane apte à apporter un ion d'hydrure lors de la réaction de réduction, et (iii) un additif enrichi de façon énantiomère sélectionné à partir du groupe consistant d'amines, de diamines, d'alcools, de diols, d'acides organiques, de diacides organiques, de thiols et de phosphines;
de réaction d'un substrat de cétone en présence du mélange; et
la récupération et l'épuration d'un produit de réaction d'alcool enrichi en un énantiomère.

26. Le procédé à la revendication 25 selon lequel le catalyseur est sélectionné à partir du groupe consistant d'alkoxydes métalliques et d'aryloxydes métalliques.

27. Le procédé à la revendication 26 selon lequel ledit catalyseur est sélectionné à partir du groupe qui consiste d'isopropoxyde de titane (IV), d'isopropoxyde de trichloro-titane (IV), d'éthoxyde de titane (IV), de méthoxyde de niobium (V), et de butoxyde de titane (IV).

28. Le procédé à la revendication 25 selon lequel le composé de silane est sélectionné à partir du groupe qui consiste de silane, diphényle silane, phényle silane, diéthyle silane, dinéthyle silane et triéthoxy silane, triméthoxy silane et poly(méthyle hydrosiloxane).

29. Le procédé à la revendication 25 selon lequel l'additif est présent en un montant entre 0,1 et 100 moles pour cent, par rapport au montant de substrat.

30. Le procédé à la revendication 29 selon lequel l'additif chiral est sélectionné à partir du groupe consistant de (1R,2R)-diamino cyclo-hexane; (1S,2S)-diaminocyclohexane; (R)-1,1'-Bi-2-naphtole, (S)1,1'-Bi-2-naphtole; (1R,2S)-éphédrine; (1S,2R)-éphédrine; et 1,1,4,4-tétraphényle-2,3-0-isopropylidène-D-threitol.
